(19) Europäisches Patentamt
European Patent Office
Office européen des brevets

(11) EP 3 990 620 B1

(12) **EUROPEAN PATENT SPECIFICATION**

(45) Date of publication and mention of the grant of the patent:
**13.12.2023 Bulletin 2023/50**

(21) Application number: **20743589.2**

(22) Date of filing: **29.06.2020**

(51) International Patent Classification (IPC):
***C12N 5/00*** *(2006.01)* ***C12N 5/079*** *(2010.01)*
***C12Q 1/02*** *(2006.01)*

(52) Cooperative Patent Classification (CPC):
**C12N 5/0622; C12N 5/0619; C12N 5/0697;**
C12N 2502/081; C12N 2502/086; C12N 2502/28;
C12N 2503/00; C12N 2533/50

(86) International application number:
**PCT/EP2020/068264**

(87) International publication number:
**WO 2020/260697 (30.12.2020 Gazette 2020/53)**

(54) **IN VITRO CELL CULTURE SYSTEM UNDER AEROBIC CONDITIONS SUITABLE FOR PHYSIOLOGICAL, PATHOLOGICAL, PHARMACOLOGICAL AND TOXICOLOGICAL INVESTIGATIONS**

IN-VITRO-ZELLKULTURSYSTEM UNTER AEROBEN BEDINGUNGEN FÜR PHYSIOLOGISCHE, PATHOLOGISCHE, PHARMAKOLOGISCHE UND TOXIKOLOGISCHE UNTERSUCHUNGEN

SYSTÈME DE CULTURE CELLULAIRE IN VITRO DANS DES CONDITIONS AÉROBIES APPROPRIÉES POUR DES RECHERCHES PHYSIOLOGIQUES, PATHOLOGIQUES, PHARMACOLOGIQUES ET TOXICOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2019 IT 201900010428**

(43) Date of publication of application:
**04.05.2022 Bulletin 2022/18**

(73) Proprietor: **Armato, Ubaldo**
**37059 Zevio (IT)**

(72) Inventors:
- **ARMATO, Ubaldo**
  **37059 Zevio (IT)**
- **DAL PRÀ, Ilaria Pierpaola**
  **37059 Zevio (VR) (IT)**
- **CHIARINI, Anna Maria**
  **25018 Montichiari (BS) (IT)**

(74) Representative: **Calogero, Ida et al**
**BIRD & BIRD SOCIETA TRA AVVOCATI S.R.L.**
**Via Porlezza, 12**
**20123 Milano (IT)**

(56) References cited:
- **WENDT H ET AL: "Artificial Skin - Culturing of Different Skin Cell Lines for Generating an Artificial Skin Substitute on Cross-Weaved Spider Silk Fibres", PLOS ONE, vol. 6, no. 7, E21833, 26 July 2011 (2011-07-26), XP055299717, DOI: 10.1371/journal.pone.0021833**
- **LIU J ET AL: "Silk Fibroin as a Biomaterial Substrate for Corneal Epithelial Cell Sheet Generation", INVESTIGATIVE OPTHALMOLOGY & VISUAL SCIENCE, vol. 53, no. 7, 25 June 2012 (2012-06-25), pages 4130-4138, XP055675388, ISSN: 1552-5783, DOI: 10.1167/iovs.12-9876**
- **WANG S ET AL: "In vitro3D corneal tissue model with epithelium, stroma, and innervation", BIOMATERIALS, vol. 112, 4 October 2016 (2016-10-04), pages 1-9, XP029812696, ISSN: 0142-9612, DOI: 10.1016/J.BIOMATERIALS.2016.09.030**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- GOSSELIN E A ET AL: "Multi-layered silk film coculture system for human corneal epithelial and stromal stem cells", JOURNAL OF TISSUE ENGINEERING AND REGENERATIVE MEDICINE, vol. 12, no. 1, January 2018 (2018-01), pages 285-295, XP055675394, ISSN: 1932-6254, DOI: 10.1002/term.2499
- GUPTA A K ET AL: "Scaffolding kidney organoids on silk", JOURNAL OF TISSUE ENGINEERING AND REGENERATIVE MEDICINE, vol. 13, no. 5, 20 May 2019 (2019-05-20), pages 812-822, XP055675397, ISSN: 1932-6254, DOI: 10.1002/term.2830


Remarks:

The complete document including Reference Table(s) and the Sequence Listing(s) can be downloaded from the EPO website

## Description

### FIELD OF THE INVENTION

The invention concerns a method and a new *in vitro* cell culture system, wherein the cells, preferably isolated from the human central or peripheral nervous system, but also from any other human tissue, are incubated on a new conception photo-bioactive hybrid support situated at the interface between the air and the nutrient medium. Such a system allows the cells first to proliferate and then to differentiate and perform functional activities typical of their mature phenotype under optimal aerobic and nourishing conditions.

The use of the system for the screening of pharmaceutical, genotoxic and neurotoxic compounds and for *in vitro* cell culture is also described.

### STATE OF THE ART

The human nervous system is the most complex neurological structure found in the terrestrial globe, so complex to be very different from that of the vast majority of other mammals, with the partial exception of great anthropomorphic apes. These unique morpho-functional characteristics of the human brain are the basis of our species' pre-eminence over all the others.

In recent decades, medical advances have led to a substantial extension of the average human life span. At the same time, this extension led to a surge in the prevalence of diseases, mostly degenerative, affecting the human central nervous system (CNS). Morbid forms once considered a rarity, e.g. neurodegenerative diseases [see Alzheimer's disease (AD)], have seen their prevalence grow dramatically while they still totally lack for effective therapies that suppress or mitigate their triggering etiological factors which remain, *inter alia,* unknown or object of intense diatribes. One of the reasons for the protracted lack of specific therapies for human neurodegenerative diseases lies in the fact that the CNS of laboratory animal models, in particular rodents, and the human CNS are very different in terms both of morphological and functional profiles. Other organs, e.g. the heart, are morpho functionally similar, and therefore more useful for identifying potentially pathological mechanisms and possible therapies thereof. Unfortunately, this does not apply to CNS. Rodent models for human Alzheimer's disease (AD), although of some usefulness in terms of general neurobiology, have not allowed to ultimately identify even a single therapy which, once transferred to patients, was able to usefully influence the course of the disease. The same happened in the case of other human CNS diseases. On the other hand, anthropomorphic apes are very expensive, difficult to manage, they belong to protected species, and the course of CNS diseases, particularly chronic ones, is very slow in them and takes many years like in humans. In addition, human CNS diseases, especially chronic ones, often exist in more than one form or variant. For example, there are two different etiological forms of AD:

(a) Familial Early-Onset Alzheimer's Disease (fEOAD), i.e. starting before age 60, and (b) Sporadic Late-Onset Alzheimer's Disease (SAD/LOAD) which comprises 96-97% of cases, occurs after 65-70 years, develops more slowly, and is caused by multiple etiological factors of a still debated nature. As already specified, animal models in rodents do not reproduce SAD/LOAD at all. The information available so far on SAD/LOAD derives either from autopsy findings or from clinical observations on patients, from the results of investigations carried out on the SNC using PET (positron-emission tomography) and MRI (magnetic resonance imaging) techniques, and from the analysis of specific protein levels (Aβ42, Aβ40, total Tau, hyperphosphorylated Tau) in the liquor (or cerebrospinal fluid, CSF). These methods are not apt to perform experimental investigations and are little suitable for epidemiological investigations since they are rather expensive and inherently technically difficult to execute, and it is therefore not easy to use them for extensive or longitudinal investigations on the disease natural course or the efficacy of potential drugs. The foregoing on fEOAD and SAD/LOAD also applies to other diseases, especially chronic diseases, that affect the human CNS. Taken together, these considerations clearly indicate the need to develop *in vitro* preclinical models of the aforementioned diseases providing experimental results that can be transferred more successfully to clinical settings. In the past, many attempted to use tumour cells isolated from the human CNS (astrocytomas, gliomas, neuroblastomas, etc.) for setting up these *in vitro* preclinical models as they are not technically difficult to handle. However, in normal conditions, once the extrauterine life has started, the proliferative activity of human brain cells is almost completely extinguished and the cells undergo important maturation processes whereby their transcription, translational and metabolic phenotypes are profoundly different from those of their transformed counterparts. In other words, the information obtained from tumour nerve cells relate almost only to the characteristics of these latter ones. Other considerations that should not be underestimated concern the considerable technical difficulties encountered when trying to culture *in vitro* normal (non-transformed) nerve cells of the human CNS. Furthermore, still regarding the latter, there are several questions that need to be answered in advance. A first question is whether or not mixed models should be set up, in which there are multiple types of previously purified CNS cells

and in a defined composition, e.g. neurons and astrocytes, neurons and oligodendrocytes, neurons and microglia, and so on, or whether mixed models that have not undergone any prior purification should be preferred. Or alternatively, whether monotypic cultures, such as neurons only or astrocytes only, etc., are to be set up and studied, after specific purification. Monotypic cultures are preferable in the initial stages of studies in which the type-specific cellular responses are not at all or very little known. Another issue to keep in mind is the site of origin of the CNS or PNS nerve cells as it significantly impacts the morphofunctional characteristics of the same cells.

A further, even more important question than the previous ones is the following: what certainties do we have that the *in vitro* model allows nerve cells to function as or almost as they function *in vivo?* It is well known that human nerve cells, in particular neurons, are eager for oxygen ($O_2$). The standard *in vitro* cultures currently in use provide that cells are submerged by a growth medium whose thickness can be from 5 to 20 mm or more. In such situations of deep immersion, however, the cultured nerve cells are exposed to a persistent anaerobic condition, i.e. relative deficiency of $O_2$, which is directly proportional to the thickness of the liquid above the cells, and considerably limits their basal metabolic activities, the responses to physiological and/or pathological stimulating agents and, in the case of neurons, also the *in vitro* survival capabilities. As known to those skilled in the art, but generally ignored or tacitly forgotten, this is due to the fact that oxygen ($O_2$) is very little water soluble: if it were water soluble, it would not be necessary to have 13 to 18 grams of haemoglobin (Hb) enclosed in red blood cells per 100 mL of circulating blood in order to convey sufficient amounts of $O_2$ to our body tissues. On the other hand, if added to the culture medium, free Hb is cytotoxic and therefore cannot be used in this form to reduce hypoxia in cultured cells.

Therefore, the increase in the prevalence of human CNS diseases and the difficulty of studying the specific metabolic characteristics of the cells that constitute it, both in physiological and pathological conditions, are among the most important reasons for the persistent lack of truly effective therapies for the same diseases.

The object of the present invention is a method and a new *in vitro* cell culture system that allow to experimentally study the metabolic characteristics of the different types of human CNS cells, either in basal or physiological conditions, and in conditions that simulate the diseases of this organ and allow also to identify new etiological therapeutic approaches, i.e. capable of blocking or significantly mitigating the crucial upstream molecular processes that determine the symptoms and downstream hallmarks of these neuropathologies.

**SUMMARY OF THE INVENTION**

In light of the above, with the present invention a new platform which solves the difficulties listed above and allows to experimentally study the metabolic characteristics of the different types of human CNS cells in both normal and pathological conditions is provided. This system is valid whether it constitutes a model of cell physiology or it represents an *in vitro* preclinical model ("in a Petri dish") of Alzheimer's disease or other acute or chronic human CNS diseases.

The invention therefore concerns a method for cell culture *in vitro,* comprising the steps of:

**a.** inserting a support for the cell growth and maintenance in a cell culture container;
**b.** putting one or more cell types in contact with and allowing to attach to the support for the cell growth and maintenance of step **a.;**
**c.** placing the support with the attached cells of step **b.** to float at the interface between the air and the culture medium;
**d.** incubating the floating support on the culture medium of step **c.** in appropriate conditions that allow the growth and then differentiation and good functioning of the cells;

wherein:

- said one or more cells grow at the interface between the air and the cell culture medium, and exchange nutrients and metabolites with the underlying growth medium, and
- said support is a fibroin microfiber textile tissue integrated with a fibroin film.

Said support for the growth and subsequent functional maintenance of the cells floats by itself on the culture medium and, being permeable to gases, allows on one hand active exchanges of $O_2$ and $CO_2$ between the adherent cells and the air above and, on the other hand, exchanges of metabolites and catabolites between the adherent cells and the underlying growth medium with which they are in contact.

In a further aspect, the present invention concerns a cell culture system comprising an integrated combination of a fibroin microfiber textile tissue and a fibroin film.

In yet another aspect, the present invention describes a use of the system for the screening of pharmaceutical, genotoxic or neurotoxic compounds.

In still another aspect, the present invention describes a use of the system for *in vitro* cell culture of neurons, astrocytes, oligodendrocytes, microglia cells or cerebrovascular apparatus cells preferably derived from the human nervous system

## DESCRIPTION OF THE FIGURES

The invention will now be described in detail and with reference to the attached figures in which:

**Figure 1** shows the cell culture system, and in particular the structure of the fibroin photo-bioactive hybrid support for *in vitro* cell cultures object of the present invention seen under an optical microscope at a magnification of 100X. The two components are clearly distinguished, the yarn net consisting of large mesh fibroin microfiber cords which is incorporated and integral with the fibroin film covering also the spaces delimited by the yarn/textile net.

**Figure 2** highlights the fluorescence of fibroin illuminated by a LED light having a wavelength (λ) of 608 nm and the lack of fluorescence of the same when in the dark.

**Figure 2A.** *Top.* When not illuminated, the fibroin photo-bioactive hybrid support for *in vitro* cell culture does not emit any fluorescence, as it can be expected.

**Figure 2B.** *Bottom.* When illuminated with a red LED lamp (λ = 650 nm) the fibroin photo-bioactive hybrid support for *in vitro* cell culture emits a green fluorescence that is weak at the areas where there is only the fibroin film but it is more intense at the structures of the textile / yarn of fibroin microfibers incorporated in the same film.

This secondary fluorescence obtained with short-term illumination (around 10 minutes per day) has beneficial effects on the metabolism of cells attached to the photo-bioactive hybrid support placed to float on the growth medium at the interface between it and the air.

**Figure 3** highlights the photo-bioactive hybrid support of silk fibroin which floats on the growth medium contained in a plastic cell culture tray. The weft of the yarn, which is integrated in the fibroin film, can be distinguished. Cells attached to the underlying surface of the photo-bioactive hybrid support are not visible.

**Figure 4:** At 8 hours from seeding, human astrocytes intravitally stained with the lipophilic tracer DiOC18(3) (Thermo Fisher Scientific), which binds to the cell membrane phospholipids, have attached to the fibroin photo-bioactive hybrid support floating at the interface between the atmospheric air and the growth medium. Part of the microscopic field is out of focus due to the particular structure (textile yarn + film) of the photo-bioactive hybrid support. Original magnification: 100X.

**Figure 5:** Sectional view schematic drawing of a cell culture container (a) containing the culture medium (b) and the photo-bioactive hybrid support for cell cultures (d) with attached cells (e). The photo-bioactive hybrid support (d) has the surface (c) in contact with the air. Measurements are not to scale.

**Figure 6:** Perspective view schematic drawing of the photo-bioactive hybrid support for cell cultures (d) having the cells (e) attached to the surface (c) which is placed in contact with the culture medium. Measurements are not to scale. The magnification (a) shows the cells attached to the surface (c) of the photo-bioactive hybrid support.

**Figure 7:** Comparison of the cell numbers of nontumorigenic cortical adult human astrocytes grown for a week on silk fibroin (SF) films, SF textile fibers, and on integrated SF textile plus film structures, respectively. It is evident that the integrated structures support a significantly more intense astrocyte growth than each of the separate components. Bars are means ± SEMs of three distinct experiments, each performed in duplicate. *, $P < 0,05$ vs. SF film. **, $P < 0.05$ vs. SF textile fibers.

## DETAILED DESCRIPTION OF THE INVENTION

The present invention therefore concerns a method for *in vitro* cell culture, comprising the steps of:

**a.** inserting a support for the cell growth and maintenance in a cell culture container;

**b.** putting one or more cell types in contact with and allowing to attach to the support for the cell growth and maintenance of step **a.;**

**c.** placing the support with the attached cells of step **b.** to float at the interface between the air and the culture medium;

**d.** incubating the floating support on the culture medium of step **c.** in appropriate conditions that allow the growth and then differentiation and good functioning of the cells;

wherein:

- said one or more cell types grow at the interface between the air and the cell culture medium, and exchange nutrients and catabolites with the underlying growth medium, and
- said support is a fibroin microfiber textile tissue integrated with a fibroin film.

In one embodiment of the method according to the present invention, said one or more cells first grow and remain viable, and then differentiate and remain functional at the interface between the air and the cell culture medium, and exchange nutrients and catabolites with the underlying growth medium.

In a preferred embodiment, said support for the cell growth and maintenance is a photo-bioactive hybrid support.

In the present invention when the following definition is used:

- "floats" or "floating" means to remain suspended on the surface of the culture medium, to float or be only to a limited extent immersed in the culture medium in order to keep the cells at the interface or at a distance of less than 1.2 mm from the atmospheric air, and therefore exposed both to a partial pressure of $O_2$ equal to or very close to atmospheric pressure as well as to the nutrients contained in the growth medium;
- "photo-bioactive hybrid support" means a cell culture system comprising a fibroin microfiber tissue (also indicated as yarn) integrated with a fibroin film, capable of absorbing photons emitted by LED lamps of any colour and emitting fluorescent light that is absorbed by the cells that are attached to it, whose metabolic processes and proliferative activity are accelerated.

The method of the present invention employs a system for the cell growth comprising an integrated combination of a fibroin tissue and a fibroin film (Figure 1) which allows the study of the different types of cells, preferably cells of the human CNS both in basal conditions (physiological) and in pathological conditions. By using the method and the photo-bioactive hybrid support of silk fibroin (silk fibroin or SF) according to the present invention and cells cultured therewith, all the diseases affecting the human central nervous system and peripheral nervous system can be reproduced and studied *in vitro,* pharmacological remedies for them can be identified and, finally, it is possible to define if a chemical compound, already in use or newly synthesized, is genotoxic and/or neurotoxic for humans or not.

The present *in vitro* cell culture method solves some problems encountered with the classic *in vitro* preclinical systems in which the cells are deeply immersed in the culture medium. In these standard methods, human cells, as well as those of animal origin, are covered by a growth medium thickness in the order of 2-20 mm. Since $O_2$ by its very nature is not very water soluble, its partial pressure or tension drops rapidly as soon as the thickness of the medium covering the cells exceeds the value of 1.34 mm (the latter is the thickness that can be used only for a maximum of 3-4 hours at 37 °C as it undergoes a progressive evaporation with consequent drying and death of the cells). With thicknesses of the medium above the cells greater than 1.34 mm, cells are cultured in anaerobiosis or deep and permanent $O_2$ deficiency, so the energy (amount of consumable ATP) available to them, that reflects their metabolic capacities, is reduced to even less than 1/10 of what they would have available under aerobic conditions.

In particular, it is possible to overcome the aforementioned biological problems in the *in vitro* culture of non-transformed (i.e. non-neoplastic) cells isolated from the human CNS, as their well-known and considerable needs of $O_2$, and therefore their metabolic capacities, are guaranteed to normal levels by the *in vitro* culture system according to the present invention.

Advantageously, in a preferred embodiment of the method according to the present invention, said photo-bioactive hybrid support for cell growth comprises silk fibroin produced by *Bombyx mori* or by a wild moth, such as *Antherea pernyi, Antherea yamamai, Antherea mylitta, Antherea assamensis, Antherea roylei, Philosamia cynthia ricini, Aglia tau.* A preferred example of a wild moth is an *Antherea pernyi* moth. The photo-bioactive hybrid support may further comprise another biomaterial or material, preferably selected from the group consisting of a polyurethane, polyglycolic acid, polylactic acid, chitosan, collagen, gelatin, cellulose, fibronectin, hyaluronic acid, and combinations thereof. These polymers can improve the stability, elasticity, flexibility, biocompatibility and optical and mechanical properties of the silk fibroin photo-bioactive hybrid support.

Furthermore, the aforementioned photo-bioactive hybrid support systems for cell cultures may vary in their respective per cent formulations and in the degree of crystallization of their basic constituent material, i.e. fibroin or SF. These systems are able to absorb the photons emitted by LED lamps of any colour and to emit fluorescent light that is absorbed by the adherent cells, thus accelerating the metabolic processes and proliferative activity thereof. Preferably, the LED lamp emits a green ($\lambda$ = 550 nm) or red ($\lambda$ = 650 nm) (Figure 2) or near infrared ($\lambda$ = 808 nm) light; two or all three of the lamps mentioned above can also be combined to intensify the positive biological effects thereof. The use of the aforementioned irradiation with LED lamps does not involve any increase in temperature and is not cytotoxic for cultured nerve cell within the $\lambda$ ranges mentioned.

In another aspect, a method *for in vitro* cell culture according to the present invention is described, wherein said one or more cells are human cells of any organ and tissue of the body, of the central nervous system or the peripheral nervous system. However, in other preferred embodiments, the system described herein allows to successfully culture *in vitro* any cell type of the human body, under aerobic and therefore metabolically optimal conditions. Examples of cells that can be cultured are: stem cells of all organs and tissues, cells of the lining epithelia such as cutaneous keratinocytes, transition keratinocytes with conjunctival, anal, vaginal, urethral, oral mucosa; lining epithelia of the conjunctivae, the respiratory tract (nasal cavities, nasal sinuses, nasopharynx, hypopharynx, larynx, trachea, bronchi of any size, pulmonary

bronchioles and alveoli); lining epithelia of the digestive system (oral cavity, oropharynx, oesophagus, stomach, duodenum, small intestine, large intestine, anal canal); lining epithelia of the genitourinary tract (nephrons, collecting ducts, pelvis, ureters, bladder, urethra, uterine tubae, uterus, vagina); epithelia of the exocrine glands of the integumentary, conjunctival, respiratory, digestive (including liver, gallbladder or gall bladder and exocrine pancreas) and genitourinary systems (including the prostate and seminal vesicles in men); cells of the connective-vascular structures of the conjunctival, respiratory, digestive and genitourinary systems; smooth muscle cells of the respiratory, digestive and genitourinary systems; cells of all the components of the musculoskeletal system (osteoblasts, osteocytes, osteoclasts; chondroblasts and chondrocytes; striated muscle cells and satellite muscle cells); myocardiocytes of any kind; cells of white and brown adipose tissue from any site; cells of the endocrine glands (pituitary, thyroid, endocrine pancreas, cortical portion and medullary portion of the adrenal gland; endocrine cells of the male and female gonads; other cell types of the male and female gonads, including sperm and precursors and accompanying cells thereof, and oocytes and precursors and accompanying cells thereof; spinal ganglia cells; ganglia and nerve fiber cells of the autonomic nervous system (orthosympathetic and parasympathetic) including the intramural or within the intestinal wall nervous system; peripheral nerve cells and their sheaths; epiphysis cells; meninges cells *(dura mater,* leptomeninges; *pia mater);* cells of the *tunica intima, tunica media* and *tunica adventitia* of arterial and venous vessels of any site; glomus cells; cells of the retina and of all the other components and tunics of the ocular globes and orbital cavities; crystalline cells; cells of the auditory and vestibular receptor systems; cells of the peripheral receptors at the level of joints, skeletal muscles, and integument; bone marrow cells of any type including mesenchymal stem cells; red and white cells of circulating blood; thymus cells; spleen cells; ubiquitous connective tissue cells.

In still another aspect, a method for *in vitro* cell culture according to the present invention is described, wherein said one or more cells are transformed or non-transformed human cells. Such a system may therefore also be used to culture transformed, neoplastic or tumour cells, which could be cultured using this system to test the effectiveness of chemotherapy treatments.

The method described herein therefore allows to overcome the technical problems encountered with the systems known to date. The *in vitro* cell culture methods of current use, in fact, do not guarantee at all a sufficient supply of $O_2$ to nerve cells of any type, even less if they originate from the SNC and are normal cells, that is non-transformed cells, deeply submerged by the growth medium.

In a further embodiment, a method is described wherein said one or more cells are exposed to a partial pressure of oxygen equal or close to atmospheric pressure, preferably in a range from 130 to 190 mm Hg at sea level.

It is further described an *in vitro* cell culture method according to the present invention, wherein said cell culture container is a Petri dish of various diameters (from 2.5 to 20 cm) or a plate with different number of wells (from 4 to 96).

In still another aspect, a method for *in vitro* cell culture according to the present invention is described, wherein said one or more cells are neurons, astrocytes, oligodendrocytes, microglia cells or cerebrovascular apparatus cells (pericytes, fibroblasts, smooth muscle cells, endothelial cells of cerebral vessels) preferably derived from the human nervous system.

Preferably, said cells are of non-human embryonic, fetal, postnatal or adult origin, preferably of the human cerebral cortex but also from other portions of the CNS.

The method according to the present invention is suitable for *in vitro* culture, under aerobic conditions, of any type of cells, them being either non-transformed or preneoplastic or neoplastic.

In a further aspect, the present invention concerns a cell culture system comprising a photo-bioactive hybrid support consisting of a tissue and an integrated fibroin film. Said hybrid support is an integrated combination of a fibroin microfiber textile tissue and a fibroin-film. The SF film incorporates the open structure tissue, which has "voids" closed by the SF film only between the weft and warp of the yarns.

As it can be appreciated from the photo in Figure 1, the two components can be clearly distinguished, the textile net consisting of large mesh fibroin microfiber cords which is incorporated and integral with the fibroin covering also the spaces delimited by the textile / yarn net.

Preferably, said photo-bioactive hybrid support systems for cell cultures consist of completely original configurations, for example based on silk fibroin produced by the domestic silkworm (*Bombyx mori*) or alternatively by wild moths (e.g., *Antherea pernyi, Antherea yamamai, Antherea mylitta, Antherea assamensis, Antherea roylei, Philosamia cynthia ricini, Aglia tau),* preferably *A. pernyi.* Such systems are produced by minimizing as much as possible the use of potentially neurotoxic chemical compounds that, where present, are entirely removed by the preparation method thereof. Therefore, said photo-bioactive hybrid support systems for cells are totally non-toxic and consequently have maximum biocompatibility. The portion of tissue therein represents between 20% and 60% of their weight, preferably 20-40%. They do not soak up water and thanks to their low density they completely and permanently float on the surface of the growth medium with no need of physical supports. Furthermore, these supports allow free exchange of gases (e.g. oxygen and carbon dioxide) and water vapours in the two directions, and therefore allow the so-called respiratory and metabolic processes of the attached cells.

The tissues according to the present invention may comprise different types. In a preferred embodiment, such

tissues may include flat structure tissues, such as for example orthogonal weft-warp tissues, knitted tissues, yarn or textile structures obtained by braiding and mesh structures with knot and without knot.

The second element to consider could concern the thickness of the tissue. A very wide range could be defined (e.g. from 0.01 to 2 millimetre), , then defining the optimal one as from 0.05 to 1 millimetre.

Advantageously, these tissues allow optimal cell growth since the porosity, due to the voids inside the textile weave, facilitates adhesion of the cell on the photo-bioactive hybrid support. The pores can have an area ranging from 0.0001 $mm^2$ to 25 $mm^2$, preferably from 0.0025 to 10 $mm^2$.

The photo-bioactive hybrid support for the growth and subsequent functional maintenance of the cells, according to the present invention, floats on the culture medium and being permeable to gases allows, on the one hand, active exchanges of $O_2$ and $CO_2$ between the adherent cells and the air above them and, on the other, exchanges of metabolites and catabolites between the adherent cells and the underlying growth medium.

In the cell growth and differentiation phase, said photo-bioactive hybrid support can be irradiated with LED lamps, preferably with a green light ($\lambda$ = 550 nm) but also of another colour (red: $\lambda$ = 650 nm; near infrared: $\lambda$ = 808 nm) for 10 minutes a day continuously or intermittently; this irradiation significantly increases the proliferative activity of nerve cells which therefore reach higher numbers in shorter times; the same irradiation provides an additional advantage in the cell functional differentiation phase, as they are more numerous and with a more active aerobic metabolism.

It was advantageously found that the SF film

- is the best substrate for long-term adhesion, proliferation and differentiation of human nerve cells;
- is permeable to gases (oxygen, $CO_2$) in both directions; and
- is transparent (for cell imaging, dimensional and chromatic intensity measurements thereof, etc.) and absorbs the quanta of light emitted by the LED lamp (preferably with $\lambda$ = 550 nm) or of another colour (red: $\lambda$ = 650 nm; near infrared: $\lambda$ = 808 nm) and transforms them into energy that is transferred to the adherent cells, thus increasing their mitochondrial and enzymatic function and the proliferative and differentiation activity.

The SF tissue:

- provides backing and mechanical stability to the photo-bioactive hybrid support to which the cells adhere;
- minimises the contribution to the total weight of the cell photo-bioactive hybrid support thus maintaining its floating capacity;
- maintains the flat shape of the cell photo-bioactive hybrid support, counteracting the SF film tendency to shrink when it is moistened; and
- allows the presence of large spaces consisting only of SF film where the presence and morpho-functional characteristics of the cells can be observed
- absorbs the quanta of light emitted by the LED lamp (preferably with $\lambda$ = 550 nm) or of another colour (red: $\lambda$ = 650 nm; near infrared: $\lambda$ = 808 nm) and transforms them into energy that is transferred to the adherent cells, thus increasing their mitochondrial and enzymatic function and the proliferative and differentiation activity.

Both the film and the tissue have photo-bioactive characteristics that differ, however, in intensity: the tissue has a greater intensity than the film as it contains a greater amount of fibroin per volume unit than the film.

It is therefore a unique combination of two different materials, both made of SF, which ensures optimal performance for the intended applications.

Usually, SF-based textiles (or other solid substrates) have a surface coated with a thin SF film. In this case, the solid substrate is completely integrated into the film structure.

In another preferred embodiment, these SF-based systems can slowly release into the medium growth factors such as EGF, FGF, PDGF, HGF, etc., or hormones such as insulin, glucagon, cortisol, T3/T4, oestrogens, androgens, etc., or cytokines/chemokines such as IL-1$\alpha$, IL-10, IL-6, IL-8, IL-10, IL-17, TNF-$\alpha$, IFN-y, etc., or other chemical compounds such as cholera toxin, monoclonal antibodies, etc. These compounds were previously deposited on the surface of the photo-bioactive hybrid support to which the cells will attach in the form of micro-drops of known concentration which were then dried at 37 °C. The release of the same compounds immediately after the seeding of the cells will have a targeted influence on the responses of these latter.

In another preferred embodiment, these SF-based systems can slowly release into the medium or directly to the attached cells viral or non-viral vectors of genes to be transfected into the same cells to usefully modify their metabolic and/or proliferative activities.

In a further preferred embodiment, said systems can be made of chemically modified SF to accelerate cell adhesion, or SF modified by gene mutations, or SF from other insects (*e.g.* wasps, etc.) or from arthropods (*e.g.* arachnids).

Said SF photo-bioactive hybrid support systems are easy to handle with appropriate tools (*e.g.* stainless steel

tweezers), can be cut with appropriate tools (*e.g.* punches, scalpels) in predetermined shapes and sizes (e.*g.* disks of diameters comprised between 4 and 24 mm, rectangles, rhombuses, triangles, etc.) depending on the culture plates to be used and the experimental purposes intended to be achieved (*e.g.* extraction of DNA, ribonucleic acids, proteins, transfections with viral vectors, etc.). The same supports can be sterilised in an autoclave (at 121 °C for 30 minutes) or by immersion in alcohols (methanol or ethanol for 1 hour) or by irradiation with UV lamps (30 min for each side of the supports) or with ethylene oxide, as known to those skilled in the art.

Once sterilised, said SF photo-bioactive hybrid support systems can be stored until use at room temperature or at lower temperatures for even long times (up to 5 years) in sterile containers sealed in the presence of an inert gas (nitrogen). At the moment of use, the supports are taken, placed in the culture plates (Petri dishes with or without added wells), fixed on the bottom of the same or of the wells contained in them with *ad hoc* tools (*e.g.* toroidal rings, etc., made to measure in stainless steel), and covered with a 1.2 mm-thick growth medium veil, in which the human nerve cells, of one or more selected types, to be used for the experiment were previously suspended. The surface characteristics of said SF photo-bioactive hybrid support systems allow rapid adhesion (max.: 3-4 hours) of human nerve cells. Alternatively, the side intended for the cells can be coated with a very thin layer of adhesive molecules (*e.g.* polylysine, Mussel Native Adhesive Protein [NMAP], etc.). At this point, the stainless steel tools for transient fixing to the bottom of the culture plates or wells are removed, the photo-bioactive hybrid support systems are gently extracted with a tweezers from the thin medium veil covering them, and turned upside down with a rapid movement of the wrist so that the surface to which the cells are attached is facing downwards and, finally, gently put to float on the surface of the growth medium, whose amount has in the meantime been adjusted as predetermined and whose height is now indifferent, no longer having any impact on the respiratory and metabolic capacity of cultured cells. As a result, the photo-bioactive hybrid support systems float on the surface of the medium and adherent cells are now in a doubly advantageous situation, that is, thanks to the gas permeability of the supports they are exposed both to the same (or almost) partial tension of $O_2$ (21 %) present in the atmospheric air, and at the same time they have direct access to the nutrient materials contained in the medium. In this way, cells can have a normal aerobic metabolism, exchanging $O_2$ and $CO_2$, and an equally normal usability of the nutrients contained in the medium from which to obtain chemical energy in the form of adenosine triphosphate (ATP) molecules essential for their synthetic, proliferative, secretory, and catabolic processes, etc.

Said photo-bioactive hybrid support systems for cell cultures based on SF are relatively transparent to light rays so they allow, when fresh or after intravital staining with fluorescent dyes such as DiL or DiO, or after fixation and staining according to traditional histological techniques, the observation of the adherent cells using the optical microscope in normal light, inverted or not, or phase contrast microscope, or dark field microscope, or epifluorescence microscope (with incident UV light) or confocal microscope, or using the transmission electron microscope (TEM) after specific fixation, inclusion in resin and cutting of semi-fine and fine sections at the ultramicrotome. After preparation for this purpose and metallization, it is also possible to observe the surface of the preparations and the adherent cells using the scanning electron microscope (SEM) as those skilled in the art can do.

It was advantageously verified that by the method of the present invention, nerve cells can first proliferate, stimulated not only by the availability of $O_2$/ATP but also by short (10-20 minutes) and repeated daily exposures to flashing LED lamp light, preferably green ($\lambda$ = 550 nm) but also of another colour (red: $\lambda$ = 650 nm; near infrared: $\lambda$ = 808 nm). Subsequently, nerve cells are induced to stop growing and functionally differentiate always in the presence of a correct supply of $O_2$ (around at least 90-100 mmHg of partial pressure) and nutrients while being exposed daily for 10-20 minutes to a continuous green light ($\lambda$ = 550 nm) emitted by a LED lamp. As it happens *in vivo,* the functioning of non-transformed human nerve cells, especially if deriving from critical CNS portions, such as the cerebral cortex, is deeply affected by $O_2$ supply, i.e. by the partial tension or pressure of $O_2$ in the medium or at the surface of the medium to which they are exposed.

In still another aspect, the present invention describes a use of the system for the screening of pharmaceutical, genotoxic or neurotoxic compounds.

It should be borne in mind that normal (*i.e.* non-transformed) human nerve cells isolated from the cerebral cortex can first be induced to proliferate in order to expand their number thanks to the use of appropriate growth media, and then induced to cease proliferation and undergo differentiation by switching the growth medium to another *ad hoc* medium. This second medium should be administered to said cells every 2-3 days for 2-4 weeks so that they stably acquire their phenotype and differentiated functions, and completely exit the so-called irritative phase initially induced by their permanence in the *in vitro* environment. After this phase, human nerve cells maintain for a long time their typical differentiated characteristics and constitute an experimental model with which the responses to pathological stimuli and the effects of drugs that can suppress pathological responses in a beneficial way, or the neurotoxicity of natural or synthetic chemical compounds can be tested. The use of neonatal and adult cells should be preferred as their phenotype and their functional capabilities undergo "maturation" and therefore clearly differ from those of their embryofoetal or intrauterine counterparts. They are therefore optimal systems that allow the screening of molecules that are candidates for subsequent use in human therapy.

Advantageously, the cell cultures obtainable by the method according to the present invention allow to isolate

living nerve cells from the human cerebral cortex that can be observed several times during their maintenance in *in vitro* culture and during the experimental treatments to which they are subjected without having to be necessarily sacrificed, fixed and stained for optical or electronic microscopy, unless specifically requested by the experimental protocol. This is an important aspect because it allows to use without "waste" all the cultured human nerve cells for the planned experiments while being possible to check their basic conditions, the absence of any infectious agents or toxic factors, and the effects of the different experimental treatments for their entire duration.

The cellular photo-bioactive hybrid support systems used in this method also allow (a) to replace the spent, or conditioned by the contacted cells, growth medium with fresh medium at defined times; (*b*) replace the standard medium with the medium or media containing the experimental agents to be tested (*e.g.* 0-amyloid, PFAS, etc.); (*c*) sample the same standard (control) and/or experimental media at predefined times from the start of the experiments, so as to store the samples at low temperatures (down to -196 °C in liquid nitrogen) and subsequently analyse them using molecular biology, biochemical and proteomic methods.

After taking the samples of medium, while remaining attached to the photo-bioactive hybrid support systems, the cells can be (*a*) stained and photographed to analyse and quantify their morphological modifications using *ad hoc* software packages; (*b*) treated with primary and then secondary antibodies and observed under a fluorescence or confocal microscope, and then photographed to highlight and quantify the subcellular distribution of one or more specific antigens; or (*c*) subjected to extraction of their DNA, RNA and protein contents for their subsequent analysis using *ad hoc* biochemical, molecular biology and proteomic methods.

Said photo-bioactive hybrid support systems allow to maintain *in vitro,* for a long time (up to six months) in aerobic conditions, the nerve cells of the selected type or types, isolated from the human cerebral cortex, and adhering to them. Therefore, the aforementioned photo-bioactive hybrid support systems constitute a platform that has not been available so far which is very flexible, allowing the use of the most important research and analysis methods currently available.

The method according to the present invention allows (*a*) to study the physiological aspects of normal human cortical cells [which are not necessarily identical to those of animal counterparts: the latter have a cerebral cortex that is quantitatively less developed, very differently structured (the six-layered isocortex largely prevalent in humans is very small or lacking, and does not have some of the cell types with which the human one is endowed)]; (*b*) to identify the specific molecular mechanisms that underlie the pathophysiology of the various diseases afflicting the human CNS; and (*c*) to ascertain the possible neurotoxicity of natural or synthetic (industrial) chemical compounds).

It should be adequately appreciated that the identification of the aforementioned specific pathological molecular mechanisms for human cortical cells is a prerequisite not only for understanding the natural course of each of these neurodegenerative diseases but also for identifying and testing, at a preclinical level, new etiological (or upstream) therapeutic approaches, *i.e.* acting on the mechanisms triggering and supporting the progression of the disease. So far, in fact, the therapeutic attempts for human neurological diseases, particularly neurodegenerative ones, have been only symptomatic (or downstream). Symptomatic therapies mitigate, but up to a certain point and only for a certain amount of time, the intensity of the symptoms but do not affect the course of the disease at all, and the pathological processes that promote it keep taking place undisturbed. The use of animal models (mostly transgenic) and the symptomatic therapeutic approach are the two main causes of the absence of an effective therapy for human neurodegenerative diseases. The third major cause is the lack of preclinical platforms that allow to adequately model neurological diseases by using non-transformed human nerve cells of cortical origin or other CNS origin. A striking example of this unfavourable situation is Alzheimer's disease, whose progression at the level of clinical trials on patients has never been significantly changed by any drug that had previously induced effects deemed favourable or beneficial in transgenic animal models (rodents) of the disease. The system according to the present invention adequately remedies the lack of a platform that allows to model human neurological diseases with cells of the human CNS or PNS, thus revealing their etiological molecular mechanisms and opening the way to new therapeutic treatments.

In addition to the aforementioned reasons for therapeutic failures in the treatment of CNS diseases, in particular neurodegenerative diseases, in humans, an additional factor that has long been neglected is that, despite CNS being a structure whose different cell types functionally interact in an integrated way, their loss due to pathological causes cannot be remedied (at least for the time being) through regenerative processes. For example, the loss of neurons and/or oligodendrocytes is practically definitive. Provided that in the adult CNS cells of macroglia (astrocytes and oligodendrocytes), microglia and cerebral vessels function normally, *in vivo* human neurons, *i.e.* the cells that transmit nerve impulses, can only proliferate in very restricted areas of the hippocampus, the temporal lobe and front horns of the First and Second lateral and cerebral ventricles. Some authors still question the latter possibility. Therefore, losses due to death of neurons of all the other CNS sites (almost all) are irremediable and, when they exceed a certain threshold, which corresponds to exhaustion of the so-called functional reserve (or numerical excess of cells and corresponding nerve circuits with respect to current activities), the consequent profound structural alterations cause the manifestation of the first symptoms of the disease, amnesias, that progressively worsen with the increase in specific cell loss, until reaching frank or total functional insufficiency that is clinically defined as dementia.

The present system allows to maintain and experimentally use *in vitro* differentiated neural cells that are suitable to study also etiological mechanisms in addition to physiological mechanisms, and to discover and preclinically evaluate the efficacy of new therapies for various human CNS diseases, and particularly neurodegenerative diseases. In this regard, it is also believed that the new drugs identified through said *in vitro* preclinical system should be specifically tested on patients who are in the initial asymptomatic (or silent course) phases and in the first three years of the symptomatic phases, when substantial cognitive and self-management capacities still persist.

For these same prodromal stages of human CNS diseases, the present cell culture method allows to test newly synthesised drugs or drugs previously used for other therapeutic indications (drug repurposing). These drugs will have to act on the upstream molecular mechanisms, that is strictly etiological, which will have proved crucial for the progression of human CNS diseases, in particular neurodegenerative diseases.

The system according to the present invention will allow the screening of drugs that stops or slow down significantly the progression of human CNS diseases, in particular neurodegenerative diseases. Therefore, they will allow patients, even if functionally compromised in a modest way (*e.g.* minor amnesias) to maintain their remaining endowment of neurons and cortical nerve circuits and, consequently, still sufficient mnemonic, cognitive and self-management skills. It should not be forgotten that such treatments will also lead to a significant extension and improvement in the quality of life of the patients, as well as considerable economic savings for the National Health Service of the various countries.

Another use of the *in vitro* human nerve cell systems that can be set up according to the present invention is the study of the mechanisms of action (and their relative consequences) of chemical compounds of natural or synthetic origin of current use, or the direct assessment of their possible cytotoxic effects at the level of the human cerebral cortex cells. The same platform will allow to determine the threshold levels above which the tested compounds become cytotoxic and therefore, as a practical aspect, to evaluate the actual risk of damage at the CNS level for the subjects exposed to them.

A further important use of the *in vitro* human nerve cell systems that can be set up according to the system of the present invention is the study of the mechanisms of action (and their relative consequences) of pathogens such as viruses (*e.g. human immunodeficiency virus* (HIV), cytomegalovirus, zeke, chikungunya, herpes *simplex, West Nile encephalitis,* tick-borne encephalitis, COVID-19, etc.), microbes *(e.g. Staphylococcus aureus, Acinetobacter baumanni, Listeria monocytogenes, Bacteroides fragilis, Neisseria meningitidis, Streptococcus pneumoniae* serotype 9N, *Haemophilus influenzae, Mycobacterium genus (hominis, avis* ,etc.), Gram-negative aerobic bacilli, etc.), fungi *(Fusarium sp., Cryptococcus* sp., *Histoplasma capsulatum, Blastomyces dermatitidis,* etc.), protozoa (e.g. *"human brain-eating" Acanthamoeba* spp., and *Balamuthia mandrillaris),* helminths (taeniasis, onchocerciasis, cysticercoses, etc.), and prions (Creutzfeldt-Jakob disease) that can infect or infest human CNS and, in particular, cortical cells or cells from other sites. The information in question, although apparently only of greater interest for basic research, can also have important therapeutic applications, e.g. getting to know the mechanisms by which a virus exerts cytotoxic effects on human cortical cells can help identify a drug that completely blocks them while preserving their vitality and functional capacity thereof.

Use of the system according to any one of claims 10 and 11 for the screening of pharmaceutical, genotoxic or neurotoxic compounds.

In still another aspect, the present invention describes a use of the system for the *in vitro* cell culture of neurons, astrocytes, oligodendrocytes, microglia cells or cerebrovascular apparatus cells preferably derived from the human nervous system. Said cells may be of non-human embryonic, fetal, postnatal or adult origin and of the human cerebral cortex cells. Said cells may also be transformed or non-transformed human cells.

Examples of embodiments of the present invention are provided below for illustrative purposes.

## EXAMPLES

### Example 1: Preparation of floating SF supports

The raw silk cocoons were "degummed" in an autoclave at 120 °C for 30 minutes and washed repeatedly in hot water to completely remove sericin. The pure silk fibroin microfibers (SF) were dissolved in a 9.3 M lithium bromide solution at 60 °C for 3 hours (SF concentration: 10% w/v). The SF solution was diluted 1:1 with distilled water, transferred into cellulose membrane dialysis tubes, previously washed in running water to remove glycerol and dialysed for 3 days against distilled water to remove the salts. Dialysis was stopped when the conductivity of the solution was ≤ 1 μS/cm. The aqueous SF solution was poured onto Petri dishes (diameter: 5.5 cm, 10 mL of SF solution per Petri dish) at 35 °C for at least 24 hours. Before pouring the solution, a piece of degummed silk tissue (organzine, 28 g per square meter, open structure with empty spaces of about 0.01 $mm^2$ between warp and weft threads) was deposited on the bottom of the Petri dish. After complete evaporation of the water, the composite films (SF membrane incorporating the silk tissue) were or not consolidated by immersion in an 80/20% v/v solution of ethanol/water for 1 hour, transferred into pure water for 1 hour, and finally allowed to dry at room temperature under a fume hood. The films were subsequently cut into discs of various diameters (from 4 to 24 mm) or in other geometric shapes (rectangles, squares, etc.) and subsequently sterilized at 121 °C for 30 minutes in an autoclave or by incubation for 60 minutes in 70/30 ethanol in ultrapure water.

Example 2: ***In vitro* aerobic cultures of human nerve and cerebrovascular cells**

One of the preferred formulations reported herein is the culture of astrocytes on the new SF supports. The suspension of astrocytes prepared with the method by Chiarini et al. (Int. J. Mol. Med. 2005 Nov;16(5):801-7) is divided into aliquots of $10^4$-$10^5$ cells according to predetermined amounts that are sparsely seeded on sterile SF supports, which according to a preferred formulation can be circular and have diameters ranging from 5 mm to 10 cm. The supports are then covered with a 1.5 mm thickness of the same medium. Three hours later, the SF supports together with the cells, that in the meantime have attached to them, are quickly turned upside down and immediately placed to float on the surface of the same medium that in the meantime has been brought to the desired volume. The cultures are then transferred into the incubator at 37 °C in a 95% air/5% $CO_2$ (v/v) atmosphere. Here they are irradiated for the first time in a pulsatile mode (frequency of 2.5 Hz) with a LED lamp preferably with a green light ($\lambda$ = 550 nm) or of another colour (red: $\lambda$ = 650 nm; near infrared: $\lambda$ = 808 nm) placed at a distance of 5-10 cm for a duration of 10-30 min (Repulse Lichtmedizintechnik GmbH, Austria). The lamp has a peak of radiance intensity of 80 mW/cm$^2$ and an average radiance intensity of 40 mW/cm$^2$. The same irradiation was provided every day or every other day for 8-14 days. Subsequently, the irradiation with LED lamp is continuous, lasting 5 minutes every day. In these cultures, even under aerobic conditions, astrocytes only express their specific markers such as glial fibrillary acid protein (GFAP) and glutamine synthase (GS). Other types of nerve cells and human cerebrovascular cells, isolated by appropriate methods known to those skilled in the art, express in our *in vitro* culture system under aerobic conditions and pulsatile/continuous irradiation preferably with a green LED ($\lambda$ = 550 nm) but also red ($\lambda$ = 650 nm) or in the near infrared ($\lambda$ = 808 nm) type-specific biomarkers such as enolase, neurofilament protein and neurotransmitters such as somatostatin (SST), GABA, glutamate, cholecystokinin-8 (CCK-8) and vasointestinal peptide (VIP) (neurons), galactocerebroside (oligodendrocytes), CD-68 (microglia) or factor VIII (endothelial cells), $\alpha$-actin (smooth muscle cells). These nerve cells of any type, attached to the SF floating supports, under aerobic conditions and irradiation with LED, preferably with a green light ($\lambda$ = 550 nm) but also red ($\lambda$ = 650 nm) or in the near infrared ($\lambda$ = 808 nm) proliferate more intensely until exposure to the high calcium content medium, then differentiate according to the cell type characteristics and remain phenotypically "blocked", i.e. they stably express their typical phenotype for at least 9-12 months. At this point, the differentiated nerve cells maintained under aerobic conditions are ready to be used for experimental purposes.

**Example 3: Crucial role of Aβ●CaSR interaction on human neurons and astrocytes grown on the SF platform under aerobic conditions: induction of multiple neurotoxic effects that promote neuronal death and the spread of Alzheimer's Disease**

Untreated human cortical astrocytes and neurons are metabolically very active under aerobic conditions requiring a change of medium every two days. Their basal production of $A\beta_{42}$ is minimal and consists of monomers (which have neurotrophic activity) and very few oligomers (potentially toxic at higher levels). This picture changes radically if the proxy of $A\beta_{42}$, $A\beta_{25-35}$, is administered to human nerve cells in a soluble or fibrillary form. In fact, in the following 72 hours both astrocytes and human cortical neurons produce and release significantly greater amounts of $A\beta_{42}$ in the form of oligomers. Astrocytes treated with $A\beta_{25-35}$ also release excess amount of hyperphosphorylated Tau protein within exosomes and also excess of NO and VEGF-A, while the release of soluble neurotrophic factor (s)APP-$\alpha$ is dramatically reduced. Taken together, these neurotoxic factors produced in excess are accompanied by the slow but progressive death of human cortical neurons.

**Example 4: Cell growth rates on integrated SF textile+film, SF textile alone, and SF film alone**

Unexpectedly, some pilot experimental data suggested the possibility that the fibroin microfiber textile / yarn tissue integrated with a fibroin film could be endowed with more favourable photo-bioactive properties than was each of its two components taken alone. To further validate this hypothesis, we extended the pilot experiments performing new ones aimed at comparing in parallel the growth rates of nontumorigenic astrocytes isolated from the adult human temporal lobe cerebral cortex. To this purpose equal starting numbers (i.e. $2.0 \times 10^3$) of the astrocytes were seeded onto the SF film, the SF textile yarn fibres, and the fibroin microfiber textile tissue integrated with a fibroin film, and separately cultured *in vitro* for a week under standard conditions in a growth medium allowing their proliferation. At the end of the week, the cell numbers were determined for each group of specimens via DNA assay (Dadsetan M, Hefferan TE, Szatkowski JP, et al. Effect of hydrogel porosity on marrow stromal cell phenotypic expression. Biomaterials. 2008;29(14):2193-2202).

The results were statistically analysed via ANOVA and *post hoc* Bonferroni test. The findings proved that the growth of the astrocytes was significantly more intense on the fibroin microfiber textile tissue integrated with a fibroin film according to the present invention when compared to the growth on the SF textile fibres alone or the SF film alone (Figure 7). The data also show that the cells on the silk fibroin textile fibres were more numerous than those grown on

the fibroin film probably because the textile fibres photo-bioactive effect was more intense. Altogether, these observations indicate that the fibroin microfiber textile tissue integrated with a fibroin film exerts a more intense photo-bioactive effect on the metabolism of the human astrocytes than does each of its component by itself resulting in a greater proliferative activity of the human astrocytes under the conditions that allow it.

**Materials and Methods**

Morphological Analysis

*Cell Numbers*

The numerical densities of cells cultured on SF media were accurately assessed using the DNA Picogreen Assay. Total DNA quantification was performed using the PicoGreen DNA Detection Kit (Thermo Fisher). After a washing in 10 mL PBS for each specimen, 10 SF photo-bioactive hybrid supports are transferred into a clean tube to which 1 mL of deionized water is added to lyse the cells. Next, the samples are frozen and thawed twice and 100 μL of PicoGreen working solution is added to 100 μL of supernatant of each sample. After 2-5 minutes of incubation, the amount of DNA is measured in a fluorescence spectrophotometer (Jasco) with an excitation of 480 nm and an emission of 520 nm. The DNA amounts and the corresponding cell numbers were calculated with reference to previously constructed standard curves. It was decided not to use the popular MTT assay as it evaluates the mitochondrial activity, which is not constant but varies in different situations; and is not tightly linked to the real cell numbers and therefore can provide data not corresponding to the real situation (Quent VM et al. J Cell Mol Med 124, 1003-1013, 2010).

***Epifluorescence Microscopy***

Untreated (control) and treated neural cells attached to the SF supports were fixed for 20 minutes with paraformaldehyde (PAF; 4%) at room temperature. Subsequently, the cells were washed with 1.0 mL Tris buffer (0.05 M $L^{-1}$ Tris Buffered Saline [TBS], 9 g $L^{-1}$ NaCl, pH 8.4), four times for 5 min each time, and then permeabilised with 0.05% Triton X-100 in TBS. To saturate nonspecific binding sites, cells were incubated for 1 hour at room temperature with a blocking solution containing 2.0% FBS, 2.0% BSA and 0.02% Triton X-100 in TBS (TBS-TX). Immunofluorescence staining for $A\beta_{42}$, $A\beta_{40}$ and CaSR was performed using the respective mouse monoclonal antibodies: anti-$A\beta_{42}$ (8G7, 1.0 μg $mL^{-1}$; Acris), anti-$A\beta_{40}$ (5C3; 5.0 μg $mL^{-1}$; Acris); and anti-CaSR (HL1499, Sigma). After three washings with TBS-TX (four changes in 10 minutes), the cells are incubated with the secondary antibody conjugated with Alexa Fluor 488 used at 1.0 μg $mL^{-1}$ (Molecular Probes, Invitrogen). A 10 minute incubation with 1.0 ng $mL^{-1}$ of 4',6'-diamidino-2-phenylindole dihydrochloride (DAPI; Sigma-Aldrich) follows to stain the DNA. Coverslips are mounted with an anti-fading mounting medium (Dabco, Sigma-Aldrich). The negative control procedure omitted the primary antibody. Confocal microscopy analysis was performed on a Leica TCS SP5 AOBS (Leica-Microsystems) equipped with violet (405 nm laser), blue (Argon, 488 nm), orange (543 nm) and red (633 nm, HeNe laser) excitation laser lines. A 63X/1.40 NA oil immersion objective (PL APO 63X, Leica-Microsystem, Germany) was used for sample analysis.

Biochemical, Enzymatic and Proteomic Analysis.

***Aβ Peptides***

$A\beta_{25-35}$ (Bachem, Bubendorf, Svizzera) was dissolved at 1.5 mM in PBS. $A\beta_{25-35}$ fibrillogenesis occurred quickly (in minutes) at room temperature. Fibrillogenesis was checked by Thioflavin-T test carried out before its use in the experiments. The inverse peptide $A\beta_{35-25}$ (Bachem) used as a negative control was dissolved in the same way as $A\beta_{25-35}$, but unlike the latter it does not form fibrils. It is important to note that we used $A\beta_{25-35}$ that mimics $A\beta_{42}$ instead of the latter in order to be able to distinguish, and assay, endogenous Aβ peptides from those of exogenous origin.

***Immunoprecipitation (IP) of Human Nerve Cell Proteins***

Prior to IP, nerve cell proteins were solubilized by adding Tris-buffered saline [TBS, 20 mM Tris (pH 7.4), 200 mM NaCl, 20 mM sodium orthovanadate, and Complete EDTA-free Protease Inhibitor Cocktail (Roche)] and 1.0% NP-40. Equal amounts of protein (150 μg) were incubated at 4 °C for 2 hours with antibodies directed against specific antigens (e.g. CaSR; Santa Cruz) or caspase-3 (Chemicon-Millipore) conjugated with ImmunoPure immobilized protein A (Pierce Biotechnology, Inc., Rockford, IL). The spheres carrying the immunocomplexes were collected by centrifugation at 2000 × g for 5 minutes at 4 °C and washed five times with TBS. After a final washing, the spheres carrying the immunocomplexes were resuspended in sample buffer for immunoblot analysis.

*Two-dimensional Gel Electrophoresis (2-DE)*

Protein immunoprecipitates of nerve cells were resuspended in lysis buffer/rehydration buffer (7.0 M urea, 2.0 M thiourea, 2% w/v CHAPS, 0.5% v/v ZOOM™ Carrier Ampholytes [Invitrogen Ltd, Paisley, UK], 20 mM DTT and 0.002% bromophenol blue) for 2-DE. The proteins were first separated according to their isoelectric points (pl) on ZOOM™ strips with a 7 cm immobilized pH gradient (pl ranges from 4 to 7 and from 6 to 10; Invitrogen) and then in the second dimension according to their $M_r$ in a NuPAGE™ Novex 4-12% Bis-Tris ZOOM™ gel (Invitrogen). The gels were stained using SilverQuest™ (Invitrogen), a Silver Staining Kit compatible with mass spectrometry (MS), then scanned and the protein spot profiles were analysed with Delta2D™ software (Decodon GmbH, Greifswald, Germania).

*In-Gel Protein Digestion, MALDI-TOF/MS Analysis, PMF and Bioinformatics*

The different stages of trypsin digestion and desalination procedure in gel were performed using 96-well ZipPlate microSPE plates (Millipore) according to the manufacturer's instructions. In short, the protein spots were removed with a Spot Picker (OneTouch, The Gel Company, San Francisco, California), transferred to 96-well ZipPlate and digested overnight at 37 °C. The extracted peptides were directly applied to a target that was preloaded with a thin layer of CHCA matrix ($\alpha$-cyano-4-hydroxy-cinnamic acid, LaserBioLabs, Cedex, Francia). A Voyager De PRO™ MALDI-TOF/MS workstation (Applied Biosystems, Milan, Italy) operating in reflector mode was used. For *Peptide Mass Fingerprinting* (PMF), a 25 kV acceleration voltage was applied. The instrument calibration was performed externally using [M+H]+ ions of Des-Arg1-Bradykinin, Angiotensin-1, Glu1-fibrinopeptide B and Neurotensin. The signals corresponding to m/z values comprised between 0 and 5000 were monitored. Each spectrum was produced by accumulating data obtained with 200 consecutive laser shots. The masses present in an exclusion list containing the known peaks of the background and trypsin specific auto proteolytic peptide masses were automatically eliminated from the list of masses generated by the procedure. Data processing and protein identification via PMF were performed using the Data Explorer™ software (Applied Biosystems) and MS-Fit di Protein Prospector program (Version 5.2.2, University of California).

*Western Immunoblotting (WB)*

At previously set experimental times, the adult control human nerve cells and the treated ones attached to floating SF supports were lysed using T-PER™ buffer (Pierce-Celbio, Milan, Italy) at 4 °C containing an EDTA-free Protease Inhibitor Cocktail (Roche, Milan). The protein content of the samples was quantified according to the Bradford method by using bovine serum albumin (BSA) as a standard. Equal amounts (10-30 $\mu$g) of protein obtained from the samples were heat-denatured for 10 minutes at 70 °C in an appropriate volume of 1X NuPAGE LDS sampling buffer supplemented with 1X NuPAGE Reducing Agent (Invitrogen, Milano). The samples were then loaded on 4-12% Bis-Tris polyacrylamide NuPAGE Novex (Invitrogen) (for CaSR, $A\beta_{42}$ or for the detection of other antigens). After electrophoresis in NuPAGE MES SDS Running Buffers using Xcell SureLock™ Mini*Cell (Invitrogen; 50 min runtime at constant 200 V voltage), the proteins were transferred to nitrocellulose membranes (0.2 $\mu$m; Pall Life Sciences, Milan). To ensure an effective and reproducible binding to said membranes, the transfer was continued under low power conditions (constant voltage at 30 V) for 1 hour in NuPAGE 1X transfer buffer containing 10% methanol. The immunoblots were set up using the SNAP i.d. protein detection system (Merck-Millipore, Milan). The membranes were incubated with several primary and secondary antibodies. For example, to detect and evaluate $A\beta_{42}$ or $A\beta_{1-40}$ or CaSR the following were used: (i) the specific murine monoclonal antibody 12F4 directed against $A\beta_{42}$ obtained using a peptide corresponding to $A\beta_{42}$ C-terminal region, amino acids 37-42, coupled to Keyhole limpet hemocyanin (KLH) as a immunogen, an *in vivo* immunostimulant, through the addition of an N-terminal cysteine (Merck-Millipore); before use, the monoclonal antibody was diluted 1:300 according to seller's documentation; the 12F4 antibody does not cross-react neither with $A\beta_{1-43}$ nor with $A\beta_{1-40}$ nor with $A\beta_{25-35}$; (ii) 5C3 anti-$A\beta_{40}$ monoclonal antibody (Acris, Germania), obtained using a C-terminal peptide conjugated with KLH as a immunogen; this monoclonal does not cross react neither with $A\beta_{42}$ nor with $A\beta_{25-35}$; the 5C3 antibody was used at 1.0 $\mu$g $mL^{-1}$; (iii) specific murine monoclonal antibody HL1499 directed against CaSR (Sigma), obtained by using as an *in vivo* immunogen a peptide corresponding to the amino acids 15-29 of the extracellular N-terminal portion of CaSR; it was used at a final concentration of 3.0 $\mu$g $mL^{-1}$. An antibody directed against Lamin B1 (C-20, Santa Cruz Biotechnology, Germany) was used at 1.0 $\mu$g $mL^{-1}$ to label the loading controls. The integrated intensities of the specific bands of each protein of interest were assessed using Sigmagel™ software package (Jandel Corp., Erkrath, Germania).

*Secretase (S) Specific Activity Assay*

Treated and untreated adult human cortical neuronal cells were lysed in T-PER as described above at 4 °C. Cell lysates were centrifuged at 800 X g for 10 minutes to remove nuclei, other cell debris, and the resulting supernatant

was stored at -80 °C until further use. The respective activities of α-secretase, BACE1/β-secretase e γ-secretase enzymes were measured in the various samples of cell lysates (30-50 μg) and the results were expressed as specific activity (averages ± SEM of ΔF $\mu g^{-1}$ protein) pertaining to each experimental group. To establish the specificity of the observed γ-secretase activity, additional control assays were performed in parallel using the γ-secretase XII inhibitor (Z-IL-CHO; 8.5 μM; Calbiochem-Millipore).

***Proteasome Proteolytic Activity Assays***

Protein lysates of nerve cells (10 μg per treatment) were incubated at 37 °C with the fluorogenic substrates Succinil-Leu-Leu-Val-Tyr-AMC (SUC-LLVY-AMC; 50 μM), t-Butoxy-carbonyl-Leu-Arg-Arg-AMC (Boc-LRR-AMC; 100 μM) and Benzyloxycarbonyl-Leu-Leu-Glu-β-Naphthylamide (Z-LLE-βNA; 400 μM) (Enzo Life Sciences AG, Switzerland). The results were expressed as specific activities (average ± SEM of ΔF μg-1 protein) relative to each experimental group.

***Assay for Nitric Oxide (NO) Released by Nerve Cells in the Growth Medium***

The amount of secreted NO was inferred from the concentrations of $NO_2$ and $NO_3$, two stable oxidation products of NO, in the medium. The fluorometric method used is based on the reaction of $NO_2$ with the 2,3-diamino-naphthalene (DAN; Sigma-Aldrich) which forms the fluorescent compound 1-(H)-naphthotriazole. The nitrates in the medium are first reduced to $NO_2$ by incubation for 30 minutes with the enzyme nitrate reductase (0.1 U $mL^{-1}$). The residual NADPH was then oxidized by lactate dehydrogenase (10 U $mL^{-1}$) in the presence of 10 mM sodium pyruvate. The total $NO_2$ concentration was then determined in 50 μL of each of the cell culture supernatant samples which were brought to 100 μL with doubly deionized water. Freshly prepared DAN (10 mL of a 0.05 mg $mL^{-1}$ solution in 0.62 M HCl) was added to these diluted samples. After 10 min incubation at 20 °C, the formed naphthotriazole was measured fluorometrically at the excitation and emission wavelengths of 365 nm and 450 nm, respectively. Samples of fresh culture medium were used as blanks to correct the concentrations of $NO_2$ and $NO_3$ in the medium (~7 μM). $NO_2$ concentrations were calculated from a standard curve built using $NaNO_2$.

***ELISA Assays for A$\beta_{42}$, A$\beta_{40}$, and Soluble APP-α Secreted by Nerve cells in Samples of Conditioned Culture Media***

Quantification of $A\beta_{42}$ and $A\beta_{40}$ released by nerve cells in samples of conditioned culture media was carried out using specific, highly sensitive, Human/Rat ELISA kits, (both from Wako, Japan). The $A\beta_{42}$ kit had a dynamic range of 0.1-20.0 pM $L^{-1}$ and a sensitivity of 0.024 pM $L^{-1}$. This kit uses the monoclonal antibody BNT77, whose epitope is $A\beta_{11-28}$, and the monoclonal antibody BC05, which specifically detects the C-terminal portion of $A\beta_{x-42}$. The $A\beta_{40}$ kit had a dynamic range of 1.0-100 pM $L^{-1}$ and a sensitivity of 0.049 pM $L^{-1}$. This second kit uses the monoclonal antibody BNT77 (see above) and the monoclonal antibody BA27, which specifically detects the C-terminal portion of $A\beta_{x-40}$. Finally, a highly sensitive and specific third ELISA Kit (IBL International) was used for the assay of human soluble APP-α released in the culture medium by human nerve cells. In short, after adding a protease inhibitors cocktail (Roche), samples of the medium conditioned by human corticocerebrali cells were centrifuged for 10 minutes at 13,000 rpm to remove cellular debris. The supernatants were then tested in triplicate according to the manufacturer's protocols.

***ELISA Assay for the Evaluation of HIF-1α Transcriptional Activity***

Nuclear proteins from human cortical cells (e.g. astrocytes) were extracted using a Panomics ELISA kit (Fremont, CA) as recommended by the manufacturer. The protein content was determined using a Bradford Protein Assay Kit (Bio-Rad, Milan, Italy). Then, a Panomics ELISA kit was used to evaluate the binding of nuclear transcription factor HIF-1α to its DNA specific site (HRE). In short, to form the HIF-1α•HRE DNA complex, 40 μL of the binding buffer master mix were incubated with 10 μL of the sample nuclear extract (0.5 μg $\mu L^{-1}$) in the sample plates for 30 min at room temperature. 45 μL were transferred from each well of the sample plate to the assay plate. To capture HIF-1μ•HRE DNA complexes, the assay plate was washed three times with the buffer provided by the vendor and the samples were then incubated with a primary antibody directed against HIF-1α for 1 hour at room temperature. After washing and incubation with the secondary antibody, the colorimetric signals were developed by adding a TMB substrate solution to each well and read with a Multiskan™ Spectrophotometer (Labsystems, Helsinki, Finland) at 450 nm.

***Neurotoxicity Analysis***

Cell proliferation is an essential parameter in various fundamental researches and drug development, as it is an important process responsible for controlling the physiological and disease states in all living organisms. Generally, when exposed to compounds with different degrees of cytotoxicity, cells can react in different ways. A lethal insult causes

cell death by necrosis or apoptosis or necroptosis or by activation of autophagy processes. A sub lethal insult, on the other hand, could induce a cell proliferation inhibition and other functional alterations. Therefore, the evaluation of cell viability and proliferation is the first significant step in the toxicological studies necessary to determine pharmacological activity and safety at the cellular and molecular level. The evaluation of the cytotoxicity of a compound can be carried out using a cell viability and proliferation assay panel, which can be divided into three types:

***1. Functional Assay: Evaluation of ATP, ADP and ATP/ADP Ratio (ApoGlow, Promega).***

ATP is measured using the bioluminescence generated by the reaction based on luciferin - luciferase. This generates an amount of light measurable at a wavelength of 562 nm:

$$\text{ATP + Luciferin + } O_2 \xrightarrow{\textit{Luciferase}} \text{Oxyluciferin + AMP + PPi + } CO_2 \text{ + light (562 nm)}$$

The experiments are carried out in triplicate in 96-well luminometer plates (Wallac), with white walls and clear bottom, or in suitable supports from other suppliers (Berthold, Corning or Costar). Nucleotides are released from cell suspensions with the addition of an equal volume (in this case 100 $\mu$L) of NRR reagent. ATP levels are measured using a luminometer (Lucy 1, Anthos, Luminoskan, LabSystems or 1450 Microbeta Jet, Wallac) and expressed as the number of relative luminescence units (RLU). Under these optimal conditions and at ATP concentrations below $10^{-6}$ M, RLU is directly proportional to the amount of ATP present. The ATP signal is allowed to lapse for 10 minutes when it becomes stationary. After these 10 minutes, the ADP present in the wells is converted into ATP thanks to the addition of 20 $\mu$L of ADP conversion reagent (ADPCR). An immediate reading is performed to determine the basal RLU of the ADP (ADP 0). After the 5 minutes of incubation, necessary to allow the conversion of ADP into ATP, a third reading is carried out (ADP 5). For each well, the ADP:ATP ratio is calculated from these three readings as follows:

$$\frac{\text{ADP 5 RLU - ADP 0 RLU}}{\text{ATP RLU}}$$

The average $\pm$ SEM of the triplicates is then calculated, and the statistical significance is then assessed.

*2. Cell Membrane Damage Assay: Release of Lactic Dehydrogenase (LDH; ScienCell™* ***LDH Cytotoxicity Assay Kit)***

Lactate dehydrogenase (LDH) is a soluble cytosolic enzyme present in most eukaryotic cells that is released into the culture medium following cell death that results in damage to the plasma membrane. The increase in LDH activity in the culture supernatant is proportional to the number of cells that have been lysed. The ScienCell™ LDH Cytotoxicity Assay kit provides a colorimetric method to measure LDH activity using a reaction cocktail containing lactate, $NAD^+$, etc. LDH catalyses the reduction of $NAD^+$ to NADH in the presence of L-lactate, and the formed NADH can be measured in a coupled reaction in which the tetrazolium salt is reduced to a red product. The amount of the coloured and soluble product can be measured by a spectrophotometer at 490 nm.

*3. Mitochondrial Activity Assay: MTT (Vybrant™* ***MTT Assay Kit from Thermo Fisher)***

The MTT analysis kit represents a practical, sensitive, quantitative and reliable assay for determining mitochondrial activity in a given cell culture. This colorimetric assay is based on the conversion of MTT, a tetrazolium salt which is a pale-yellow substrate, into a formazan, a purple product. This cell reduction reaction involves the pyridine nucleotide cofactors NADH/NADPH and is only catalysed by living cells. The product has a low solubility in water and is in the form of purple crystals. The solubilisation of the resulting product with an *ad hoc* buffer allows a convenient quantification thereof. The colour intensity of the product, measured at 550-620 nm, is directly proportional to the mitochondrial activity of the cultured cells, but does not reflect their number.

***Proinflammatory Cytokines and Chemokines: Antibody Array Analysis*** *of* ***their Release***

The secretion of various cytokines/chemokines into the growth medium by nerve cells was assessed using the C-Series Human Inflammation Antibody Array (RayBiotech, USA), following the manufacturer's instructions. In particular, 1.0 mL samples of medium, which were conditioned by cells between Day 18 and Day 20 of their stay *in vitro,* were

used. After a 60 min pre-treatment with a blocking solution (Odyssey™ Blocking Buffer, LI-COR, with 0.05% Tween 20), the membrane matrices with anti-cytokine and anti-chemokine antibodies were incubated with samples of medium for 2 hours at room temperature. Subsequently, the membrane matrices were washed and incubated for 2 hours with 1.0 mL of biotin-conjugated primary antibody, diluted 1:250 in blocking solution. This was followed by an incubation at room temperature for 1 hour with 2 mL of streptavidin DyLight800 diluted 1:7,500 in blocking solution. The positive immunofluorescent signals of the cytokine and chemokine spots were acquired by the Odissey Imager™ scanner (Li-COR) and quantified using the Image Studio™ software. The intensity values resulting from each matrix were normalized to $10^3$ cells grown on the SF substrates. This type of analysis is very sensitive, up to 4-25 pg $mL^{-1}$ depending on the cytokine or chemokine considered and reveals these compounds across a concentration range of $10^4$-fold compared to the range of $10^2$ to$10^3$-fold typical of other ELISA assays. The coefficient of variation (CV) in spot intensity is 5-10%, while the CV for other ELISA assays is 10-15%.

*Molecular Biology Analysis*

***Gene Expression (mRNA) Evaluated by Reverse Transcription or RT and PCR Analysis (RT-PCR)***

The analysis of BACE1 mRNA expression (β-secretase) evaluated by RT-PCR is herein reported by way of an example. mRNAs of other genes can be analysed in a similar way as is known to those skilled in the art. Total RNA was isolated from cells using the reagent TRIzol (Thermo Fisher) and then reverse transcribed with SuperScript kit (Thermo Fisher). The cDNAs were subsequently subjected to PCR amplification using specific primers. The PCR products were separated in a 1% agarose gel containing ethidium bromide and visualised with UV light. Reverse transcription of 1.0 μg of total mRNA was performed using the cDNA Synthesis® II kit by SuperScript (Thermo Fisher), according to the vendor's instructions. Briefly, mRNA aliquots (1.0 μg) were mixed with 5X VILO™ reaction mix (1X final concentration) e 10X SuperScript® Enzyme Mix (1X final concentration). Negative controls without mRNA and without reverse transcriptase were included in each experiment. The reaction was then incubated for 10 minutes at 25 °C, for 45 minutes at 42 °C and finally for 5 minutes at 85 °C. Specific primers for human BACE1 (Sense: 5-TGCCATCTCACAGTCATCCA-3 SEQ ID NO:1; and anti-Sense: 5-TTGCCAGCCTTTTCCTTCTC-3 SEQ ID NO:2) and for glyceraldehyde-3-phosphate dehydrogenase (GAPDH) (Sense: 5-ATCACCATCTTCCAGGAGCG-3 SEQ ID NO:3; and anti-Sense: 5-CCTGCTTCACCACCTTCTTG-3 SEQ ID NO:4) were designed using Primer3 software according to the sequences published by GenBank (BACE1 accession number: NM 012104,3; GAPDH accession number: NM 002046,3). Each primer has crossed at least one exon-intron boundary. All primers have been synthesized by Invitrogen. To achieve linear amplification, the serial dilutions of cDNA preparations obtained from human cortical astrocytes were amplified by PCR using 15-30 cycles and the resulting products were purified on 2% agarose gel for 90 minutes at 90V and stained with Syto60™ (Invitrogen) 1:20,000 in RNasi-DNAsi-free water for 30 min. Standard curves were obtained for each genetic product. The reactions were carried out on a MiniCycler™ Thermal cycler (MJ Research, M-Medical S.r.l., Milan, Italy). The reaction conditions were as follows: 1X HotMaster™ Taq buffer with $Mg^{2+}$, 0.2 mM dNTPs, 0.4 μM of each primer, 0.75 U of HotMaster™ Taq DNA polymerase (Eppendorf) and 2.0 μL of 1:8 cDNA diluted for the reaction in a final volume of 25 μL. Subsequently, PCR amplifications were carried out as follows: BACE1/β-secretase: 24 cycles at 94 °C for 20 s, 58 °C for 10 s and 70 °C for 40 s; GAPDH: 22 cycles, at 94 °C for 20 s, 58 °C for 10 s, and 70 °C for 40 s, respectively. The final PCR products were subjected to electrophoresis on 2% agarose gel in 1X Tris-Borate EDTA buffer for 90 min at 90V, stained with Syto-60™ (1:20,000 in RNAse-DNase-free water) for 30 minutes, and scanned with an Odissey™ Infrared Imaging System (Li-COR Biosciences, Inc.; Lincoln, NE). The integrated intensity of each specific band was accurately quantified (in arbitrary units) using proprietary software. To allow semi-quantitative analysis, GAPDH was used as internal control. Sequencing analyses validated the identities of PCR-amplified DNA products (not shown).

***Real-Time PCR-mediated MicroRNA (miRNA) Analysis***

The levels of miRNA in human nerve cells are determined by real-time PCR analysis. Total RNA was isolated using the reagent TRIzol (Thermo Fisher) and the microRNAs were enriched using the mirVana™ miRNA Isolation Kit (Ambion). The microRNAs are reverse transcribed using the RT2 miRNA First Strand Kit from SABiosciences. The expression of miRNAs was analysed with real-time PCR using the ABI PRISM 7700 Sequence Detection System (Applied Biosystems, Foster City, CA). The small nuclear RNA U6 served as an internal control for the amplification of microRNAs. The specific primers for miRNA and U6 are obtained from SABiosciences (USA). Relative quantification of the expression levels was performed as indicated by the manufacturer's protocol.

*A8. Statistical Analysis*

***Statistical Analysis of the Results***

The data were analysed using SigmaStat 3.5™ (Systat Software) and Analyse-it™ software packages. The Shapiro-Wilk test was used to assess whether the various data groups had a normal distribution or not. The possible failure of Levene test for equality of variances and the availability of sample groups of unequal dimensions required the use of non-parametric Welch t test or Wilcoxon Z test for descriptive statistical comparisons. Parametric data were instead analysed using a one- or two-way analysis of variance (one-way or two-way ANOVA). When the ANOVA F values were significant, a Holm-Sidak *post hoc* test was used for comparisons between experimental values and untreated control values) or between values from the different treatment groups. In the case of immunoblotting, the densitometric data of the bands were normalized to the corresponding loading control bands (sheet B1) before proceeding with the statistical analysis. The null hypotheses were rejected with *values of P* < 0.05.

From the detailed description and the Examples above, the advantages achieved by the method of the present invention are apparent. In particular, this method has proved surprisingly and advantageously suitable for the *in vitro* growth and maintenance in differentiate conditions of the cerebral cortex cells of the human nervous system, under the aerobic conditions necessary to be able to reproduce the natural functional mechanisms. At the same time, this method, being relatively fast and easy to perform, can be carried out reproducibly by any person skilled in the art.

## Claims

1. A method for *in vitro* cell culture, comprising the steps of:

    a. inserting a support for the cell growth and maintenance in a cell culture container;
    b. putting one or more cell types in contact with and allowing to attach to the support for the cell growth and maintenance of step a.;
    c. placing the support with the attached cells of step b. to float at the interface between the air and the culture medium;
    d. incubating the floating support on the culture medium of step c. in appropriate conditions that allow the growth and differentiation of the cells;

    wherein:

    - said one or more cell types grow at the interface between the air and the cell culture medium, and exchange nutrients and metabolites with the underlying growth medium, and
    - said support is a fibroin microfiber textile tissue integrated with a fibroin film.

2. The method according to claim 1, wherein said support for the cell growth comprises silk fibroin produced by *Bombyx mori* or a wild moth, such as *Antherea pernyi, Antherea yamamai, Antherea mylitta, Antherea assamensis, Antherea roylei, Philosamia cynthia ricini, Aglia tau,* wherein said wild moth is preferably an *Antherea pernyi* moth.

3. The method according to claim 2, wherein said support further comprises another material, preferably a material selected from the group consisting of: a polyurethane, polyglycolic acid, polylactic acid, chitosan, collagen, gelatin, cellulose, fibronectin, hyaluronic acid and combinations thereof.

4. The method according to any one of claims 1 to 3, wherein said one or more cells are human cells of any organ or tissue of the body.

5. The method according to any one of claims 1 to 4, wherein said one or more cells are human cells of the central nervous system or the peripheral nervous system.

6. The method according to any one of claims 1 to 5, wherein said one or more cells are exposed to a partial oxygen pressure comprised in a range from 130 to 190 mm Hg.

7. The method according to any one of claims 1 to 6, wherein said one or more cells, are neurons, astrocytes, oligodendrocytes, microglia cells or cerebrovascular apparatus cells preferably derived from the human nervous system, and wherein said one or more cells are transformed or non-transformed human cells.

8. The method according to any one of claims 1 to 7, wherein said one or more cells are of non-human embryonic, fetal, postnatal or adult origin.

9. The method according to any one of claims 1 to 8, wherein said one or more cells are human cerebral cortex cells.

10. A cell culture system comprising a hybrid support, wherein said hybrid support is an integrated combination of a fibroin microfiber textile tissue and a fibroin-film.

11. The system according to claim 10, wherein said hybrid support, in which both the components, film and tissue, are photo-bioactive and said film is produced by *Bombyx mori* or a wild moth such as *Antherea pernyi, Antherea yamamai, Antherea mylitta, Antherea assamensis, Antherea roylei, Philosamia cynthia ricini, Aglia tau,* wherein said wild moth is preferably an *Antherea pernyi* moth.

12. Use of the system according to any one of claims 10 and 11 for the screening of pharmaceutical, genotoxic or neurotoxic compounds.

13. Use of the system according to any one of claims 10 and 11 for *in vitro* cell culture of neurons, astrocytes, oligodendrocytes, microglia cells or cerebrovascular apparatus cells preferably derived from the human nervous system.

14. The use according to claim 13, wherein said cells are of non-human embryonic, fetal, postnatal or adult origin.

15. The use according to claim 13, wherein said cells are human cerebral cortex cells.

16. The use according to claim 13, wherein said cells are transformed or non-transformed human cells.

## Patentansprüche

1. Verfahren für *In vitro*-Zellkultur, umfassend die Schritte

   a. Einbringen eines Trägers für das Zellwachstum und die Aufrechterhaltung in einen Zellkulturbehälter,
   b Inkontaktbringen eines oder mehrerer Zelltypen mit dem Träger für das Zellwachstum und daran anschließen lassen und Aufrechterhaltung von Schritt a,
   c Platzieren des Trägers mit den angeschlossenen Zellen von Schritt b, um an der Grenzfläche zwischen der Luft und dem Kulturmedium zu schwimmen,
   d Inkubieren des schwimmenden Trägers auf dem Kulturmedium von Schritt c. unter geeigneten Bedingungen, die das Wachstum und die Differenzierung der Zellen ermöglichen; wobei
   - der eine oder mehrere Zelltypen an der Grenzfläche zwischen der Luft und dem Zellkulturmedium wachsen und Nährstoffe und Metaboliten mit dem darunter liegenden Wachstumsmedium austauschen, und
   - der Träger ein Fibroin-Mikrofaser-Textilgewebe ist, das in einen Fibroin-Film integriert ist.

2. Verfahren nach Anspruch 1, wobei der Träger für das Zellwachstum Seidenfibroin umfasst, das von *Bombyx mori* oder wildem Falter, wie *Antherea pernyi, Antherea yamamai, Antherea mylitta, Antherea assamensis, Antherea roylei, Philosamia cynthia ricini, Agila tau,* produziert wird, wobei der wilde Falter vorzugsweise ein *Antherea* pernyi-Falter ist.

3. Verfahren nach Anspruch 2, wobei der Träger ferner ein anderes Material umfasst, vorzugsweise ein Material ausgewählt aus der Gruppe bestehend aus: einem Polyurethan, Polyglykolsäure, Polymilchsäure, Chitosan, Kollagen, Gelatine, Cellulose, Fibronektin, Hyaluronsäure und Kombinationen davon.

4. Verfahren nach einem der Ansprüche 1 bis 3, wobei die oder mehrere Zellen menschliche Zellen eines beliebigen Organs oder Gewebes des Körpers sind.

5. Verfahren nach einem der Ansprüche 1 bis 4, wobei die oder mehrere Zellen menschliche Zellen des zentralen Nervensystems oder des peripheren Nervensystems sind.

6. Verfahren nach einem der Ansprüche 1 bis 5, wobei die oder mehrere Zellen einem Sauerstoffpartialdruck ausgesetzt

werden, der in einem Bereich von 130 bis 190 mm Hg liegt.

**7.** Verfahren nach einem der Ansprüche 1 bis 6, wobei die oder mehrere Zellen Neuronen, Astrozyten, Oligodendrozyten, Mikrogliazellen oder Zellen des zerebrovaskulären Apparats sind, die vorzugsweise aus dem menschlichen Nervensystem stammen, und wobei die oder mehrere Zellen transformierte oder nicht transformierte menschliche Zellen sind.

**8.** Verfahren nach einem der Ansprüche 1 bis 7, wobei die oder mehrere Zellen nicht-menschlichen embryonalen, fetalen, postnatalen oder adulten Ursprungs sind.

**9.** Verfahren nach einem der Ansprüche 1 bis 8, wobei die oder mehrere Zellen menschliche Großhirnrindenzellen sind.

**10.** Zellkultursystem, umfassend einen Hybridträger, wobei der Hybridträger eine integrierte Kombination aus einem Fibroin-Mikrofaser-Textilgewebe und einem Fibroin-Film ist

**11.** System nach Anspruch 10, wobei der hybride Träger, in dem sowohl die Komponenten, der Film als auch das Gewebe, fotobioaktiv sind und der Film von *Bombyx mori* oder einem wilden Falter wie *Antherea pernyi, Antherea yamamai, Antherea mylitta, Antherea* assamensis, *Antherea royfet, Philosamia cynthta ricini, Agita tau,* produziert wird, wobei der wilde Falter vorzugsweise ein *Antherea* pernyi-Falter ist.

**12.** Verwendung des Systems nach einem der Ansprüche 10 und 11 für das Screening von pharmazeutischen, genotoxischen oder neurotoxischen Verbindungen.

**13.** Verwendung des Systems nach einem der Ansprüche 10 und 11 für *In* vitro-Zellkultur von Neuronen, Astrozyten, Oligodendrozyten, Mikrogliazellen oder Zellen des zerebrovaskulären Apparats, die vorzugsweise aus dem menschlichen Nervensystem stammen.

**14.** Verwendung nach Anspruch 13, wobei die Zellen nicht-menschlichen embryonalen, fetalen, postnatalen oder adulten Ursprungs sind

**15.** Verwendung nach Anspruch 13, wobei die Zellen menschliche Großhirnrindenzellen sind.

**16.** Verwendung nach Anspruch 13, wobei die Zellen transformierte oder nicht transformierte menschliche Zellen sind.

## Revendications

**1.** Procédé de culture cellulaire *in vitro,* comprenant les étapes consistant à

a. insérer un support pour la croissance et le maintien des cellules dans un récipient de culture cellulaire,
b mettre un ou plusieurs types de cellules en contact avec le support et leur permettre de s'y fixer pour la croissance et le maintien des cellules de l'étape a,
c placer le support avec les cellules attachées de l'étape b pour qu'il flotte à l'interface entre l'air et le milieu de culture,
d incuber le support flottant sur le milieu de culture de l'étape c dans des conditions appropriées permettant la croissance et la différenciation des cellules ;

dans lequel

- un ou plusieurs types de cellules se développent à l'interface entre l'air et le milieu de culture cellulaire et échangent des nutriments et des métabolites avec le milieu de croissance sous-jacent, et
- ledit support est un tissu textile en microfibres de fibroïne intégré à un film de fibroïne.

**2.** Procédé selon la revendication 1, dans lequel ledit support pour la croissance cellulaire comprend de la fibroïne de soie produite par *Bombyx mori* ou une mite sauvage, telle que *Antherea pernyi, Antherea yamamai, Antherea mylitta, Antherea assamensis, Antherea roylei, Philosamia cynthia ricini, Agila tau,* dans lequel ladite mite sauvage est de préférence une mite *Antherea pernyi.*

**3.** Procédé selon la revendication 2, dans lequel ledit support comprend en outre un autre matériau, de préférence un matériau choisi dans le groupe constitué par : un polyuréthane, l'acide polyglycolique, l'acide polylactique, le chitosane, le collagène, la gélatine, la cellulose, la fibronectine, l'acide hyaluronique et des combinaisons de ceux-ci.

**4.** Procédé selon l'une quelconque des revendications 1 à 3, dans lequel ladite ou lesdites cellules sont des cellules humaines de n'importe quel organe ou tissu du corps.

**5.** Procédé selon l'une quelconque des revendications 1 à 4, dans lequel ladite ou lesdites cellules sont des cellules humaines du système nerveux central ou du système nerveux périphérique.

**6.** Procédé selon l'une quelconque des revendications 1 à 5, dans lequel ladite ou lesdites cellules sont exposées à une pression partielle d'oxygène comprise entre 130 et 190 mmHg.

**7.** Procédé selon l'une quelconque des revendications 1 à 6, dans lequel ladite ou lesdites cellules sont des neurones, des astrocytes, des oligodendrocytes, des cellules de la microglie ou des cellules de l'appareil vasculaire cérébral, de préférence dérivées du système nerveux humain, et dans lequel ladite ou lesdites cellules sont des cellules humaines transformées ou non transformées.

**8.** Procédé selon l'une quelconque des revendications 1 à 7, dans lequel ladite ou lesdites cellules sont d'origine embryonnaire, foetale, postnatale ou adulte non humaine.

**9.** Procédé selon l'une quelconque des revendications 1 à 8, dans lequel ladite ou lesdites cellules sont des cellules du cortex cérébral humain.

**10.** Système de culture cellulaire comprenant un support hybride, dans lequel ledit support hybride est une combinaison intégrée d'un tissu textile en microfibres de fibroïne et d'un film de fibroïne.

**11.** Système selon la revendication 10, dans lequel ledit support hybride, dans lequel les deux composants, film et tissu, sont photo-bioactifs et ledit film est produit par *Bombyx mori* ou une mite sauvage telle que *Antherea pernyi, Antherea yamamai, Antherea mylitta, Antherea* assamensis, *Antherea royfet, Philosamia cynthta ricini, Agita tau,* dans lequel ladite mita est de préférence une mite *Antherea pernyi.*

**12.** Utilisation du système selon l'une quelconque des revendications 10 et 11 pour le criblage de composés pharmaceutiques, génotoxiques ou neurotoxiques.

**13.** Utilisation du système selon l'une quelconque des revendications 10 et 11 pour la culture cellulaire *in vitro* de neurones, d'astrocytes, d'oligodendrocytes, de cellules de la microglie ou de cellules de l'appareil vasculaire cérébral de préférence dérivées du système nerveux humain.

**14.** Utilisation selon la revendication 13, dans laquelle lesdites cellules sont d'origine embryonnaire, foetale, postnatale ou adulte non humaine.

**15.** Utilisation selon la revendication 13, dans laquelle lesdites cellules sont des cellules du cortex cérébral humain.

**16.** Utilisation selon la revendication 13, dans laquelle lesdites cellules sont des cellules humaines transformées ou non transformées.

Figure 1

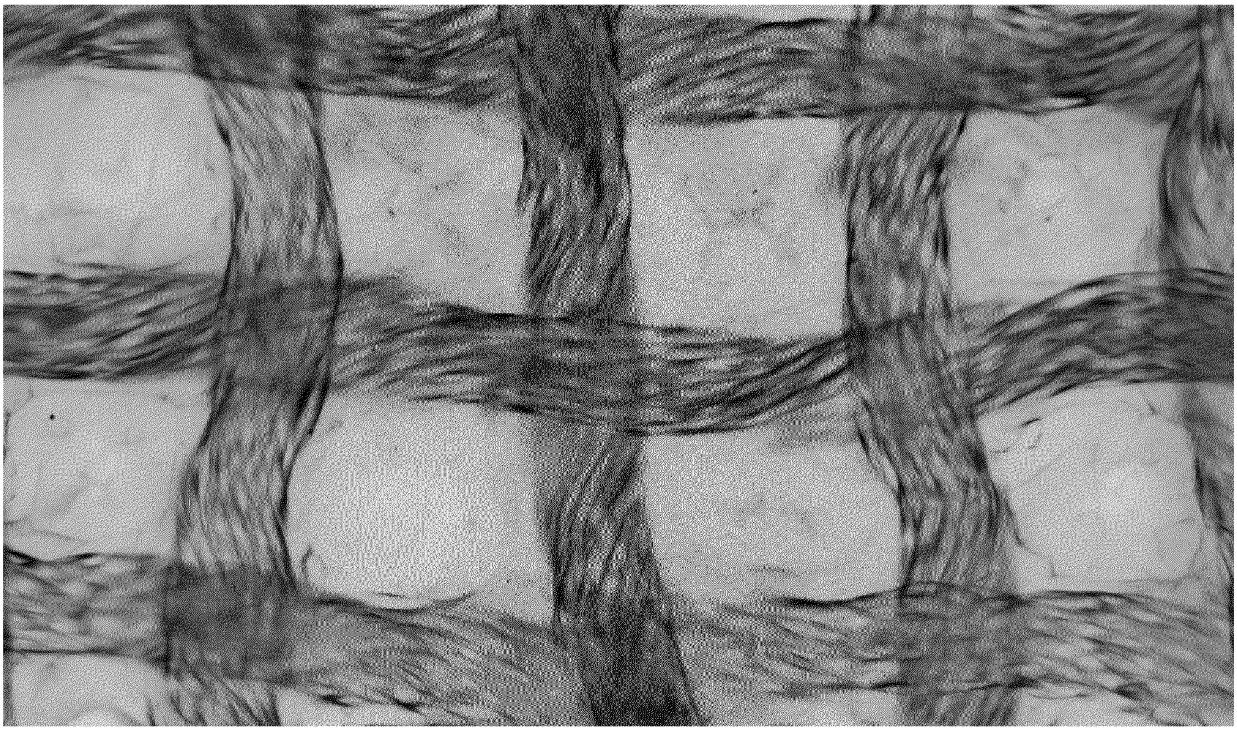

Figure 2A

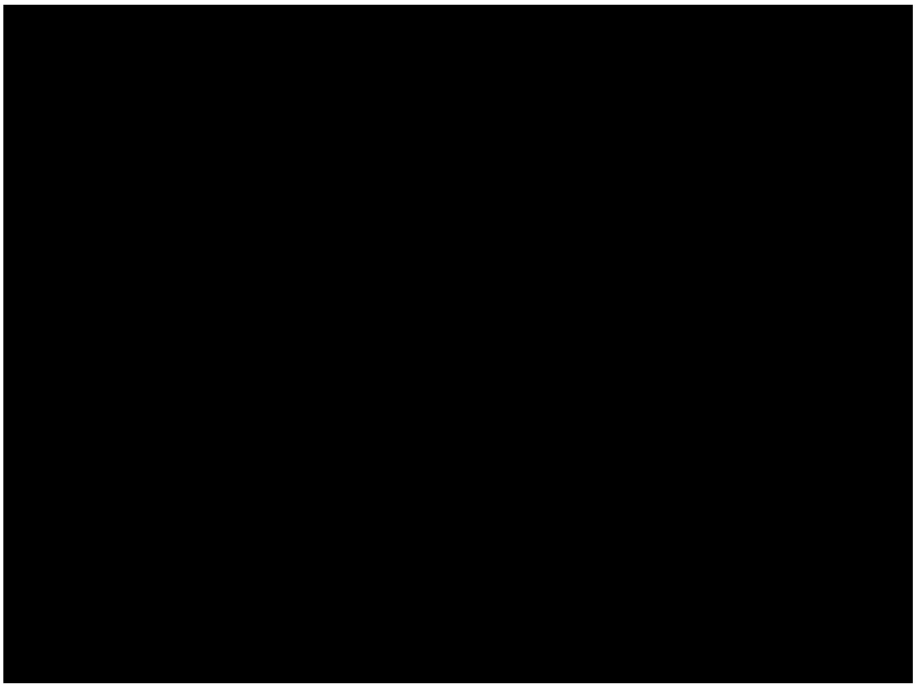

Figure 2B

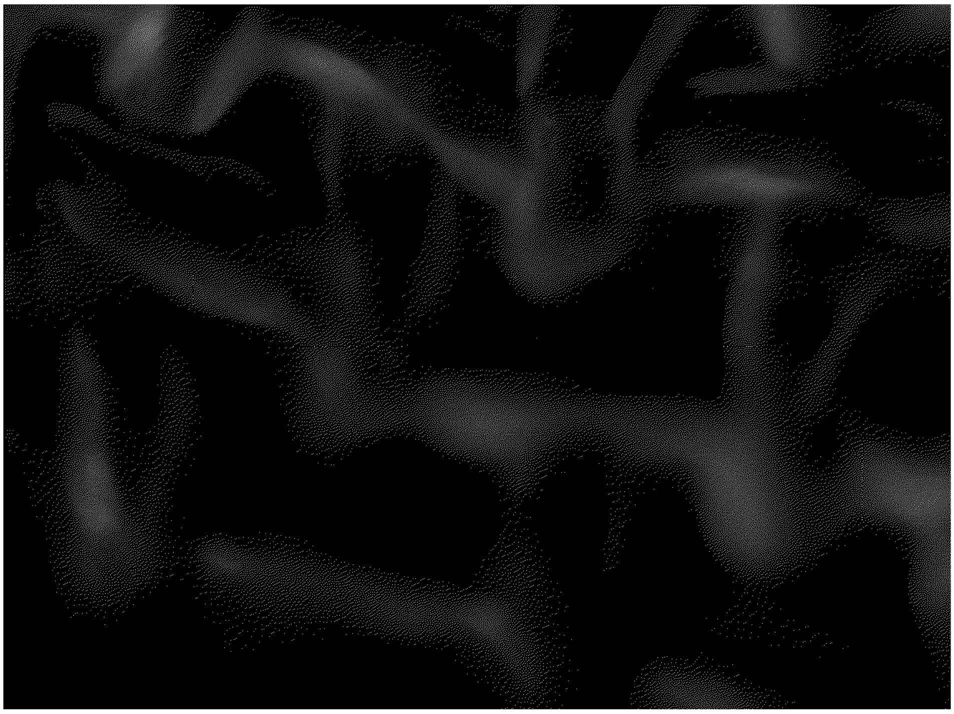

Figure 3

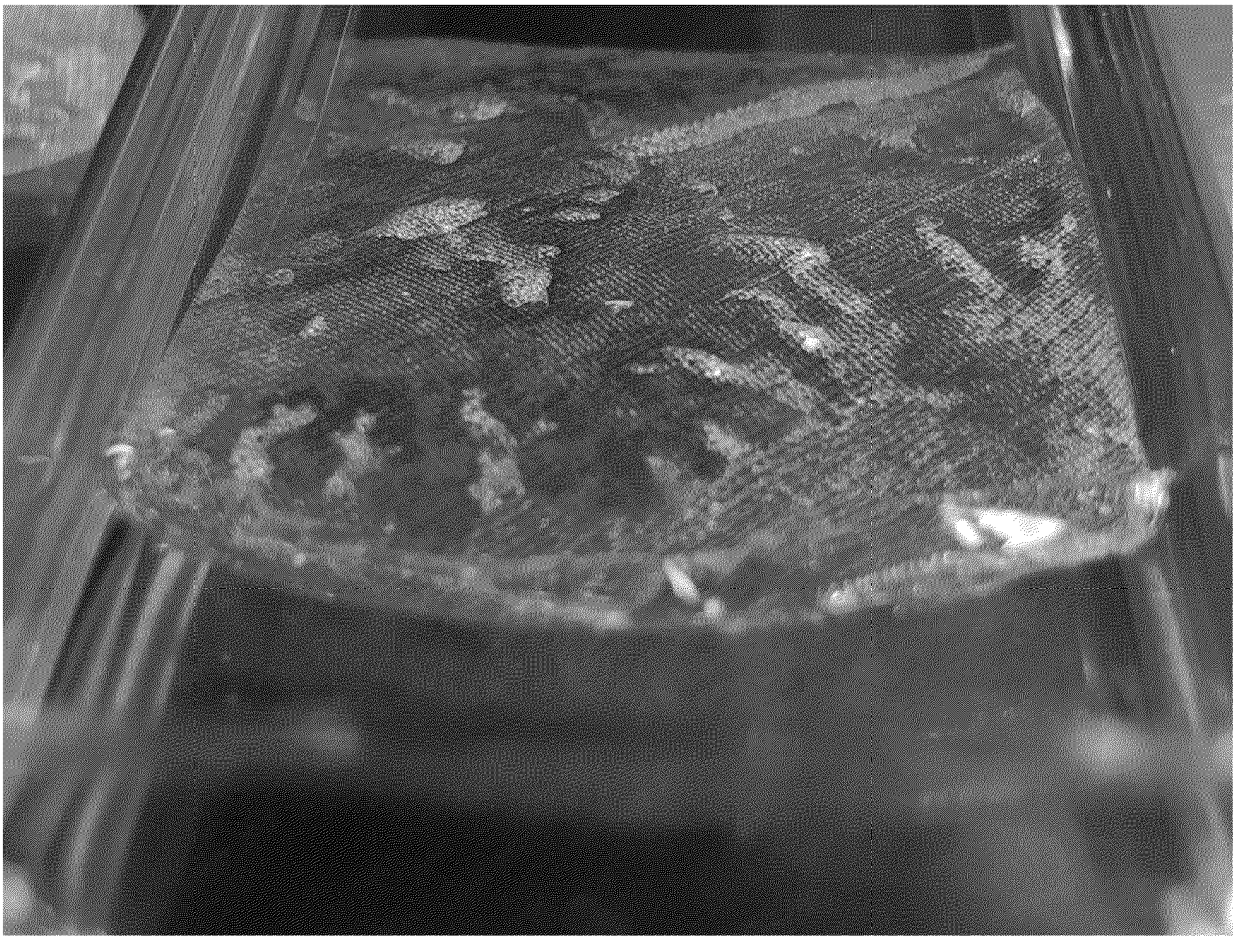

Figure 4

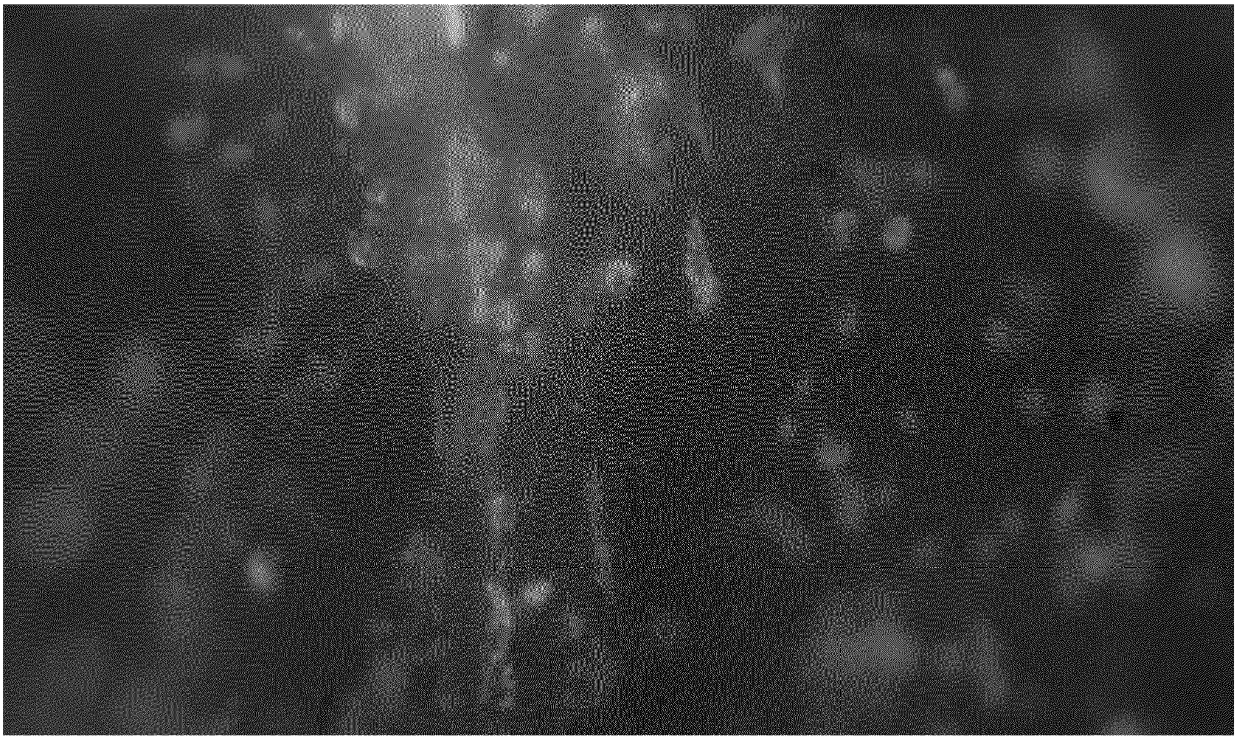

Figure 5

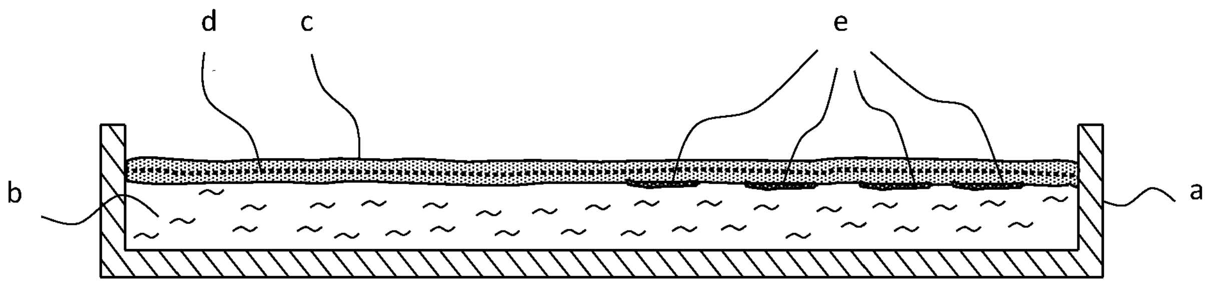

Figure 6

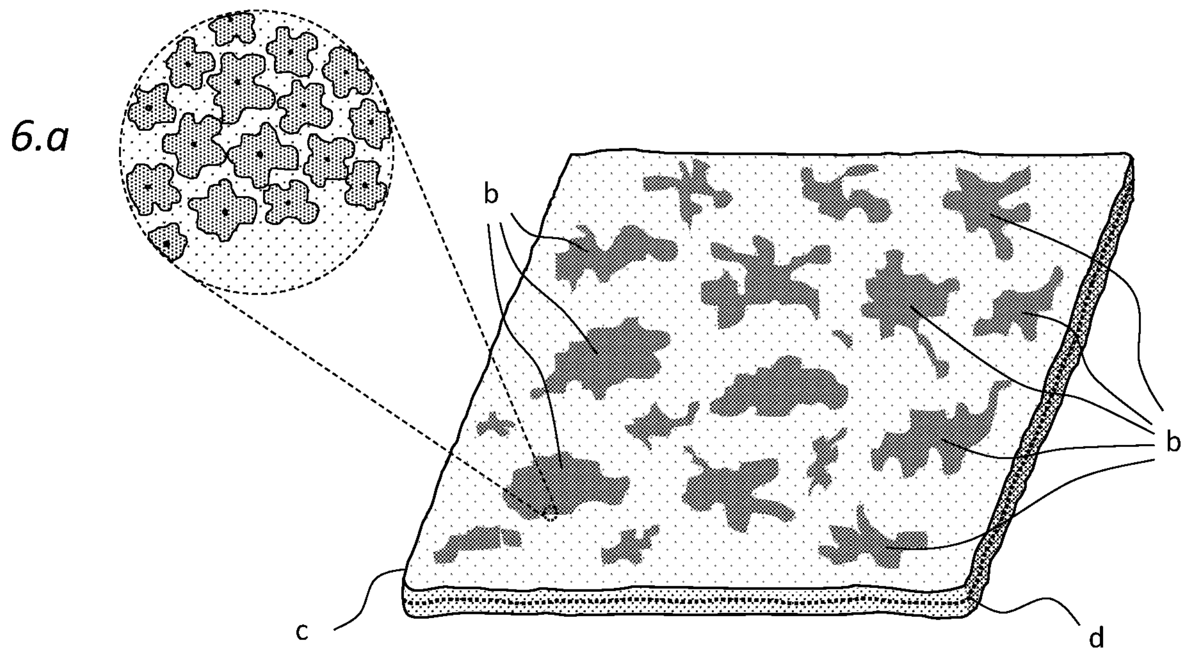

Figure 7

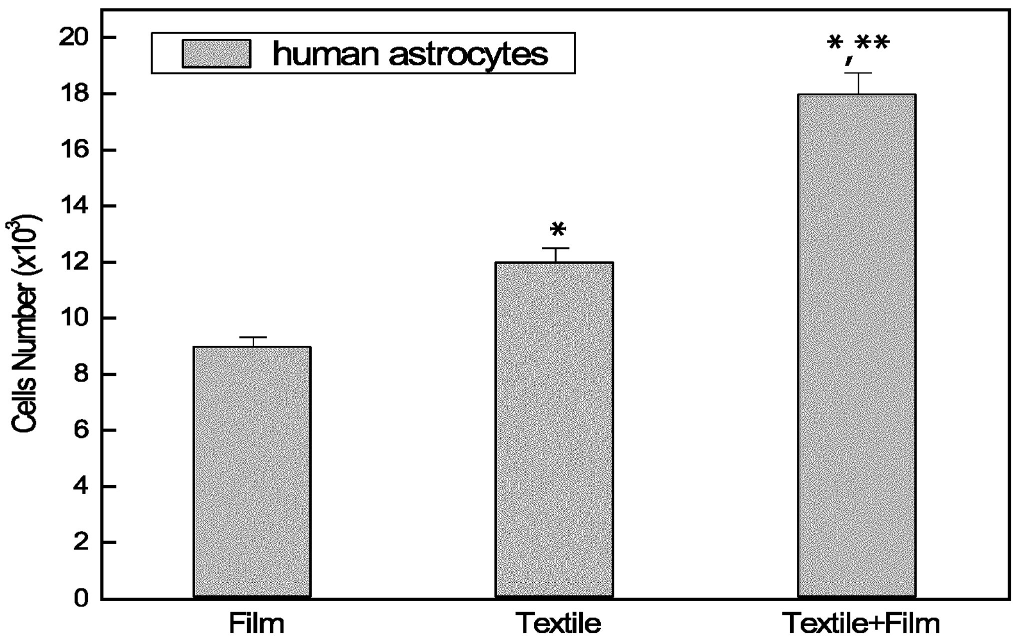
human astrocytes
Cells Number (x10³)
20
18
16
14
12
10
8
6
4
2
0
*
*,**
Film
Textile
Textile+Film

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHIARINI et al.** *Int. J. Mol. Med.,* November 2005, vol. 16 (5), 801-7
- **DADSETAN M ; HEFFERAN TE ; SZATKOWSKI JP et al.** Effect of hydrogel porosity on marrow stromal cell phenotypic expression. *Biomaterials,* 2008, vol. 29 (14), 2193-2202
- **QUENT VM et al.** *J Cell Mol Med,* 2010, vol. 124, 1003-1013
- Enzymatic and Proteomic Analysis. *Biochemical*